# EUROPEAN PATENT SPECIFICATION

(11) **EP 1 228 228 B1**
(45) Date of publication and mention of the grant of the patent: **11.07.2007**
(21) Application number: 00977111.4
(22) Date of filing: 10.11.2000
(51) Int. Cl.: C12N 15/82, C12N 15/29, C07K 14/415, A01H 5/00

(54) **SORGHUM DWARFING GENES AND METHODS OF USE**
HIRSE-SPEZIFISCHE VERZWERGUNGSGENE UND VERFAHREN IHRER ANWENDUNG
GENES NANIFIANTS DU SORGHO ET LEURS PROCEDES D'UTILISATION

(30) Priority: 12.11.1999 US 165176 P
(43) Date of publication of application: 07.08.2002
(73) Proprietor: PIONEER HI-BRED INTERNATIONAL, INC., Des Moines, Iowa 50309 (US); THE CURATORS OF THE UNIVERSITY OF MISSOURI, Columbia, MO 65211-1400 (US)
(72) Inventor: JOHAL, Gurmukh S., Columbia, MO 65203 (US); MULTANI, Dilbag S., Urbandale, IA 50322 (US)
(74) Representative: Bentham, Andrew
(86) International application number: PCT/US2000/030816
(87) International publication number: WO 2001/034818

(56) References cited:
- DUDLER R ET AL: "STRUCTURE OF AN MDR-LIKE GENE FROM ARABIDOPSIS-THALIANA EVOLUTIONARY IMPLICATIONS" JOURNAL OF BIOLOGICAL CHEMISTRY, vol. 267, no. 9, 1992, pages 5882-5888, XP002925139 ISSN: 0021-9258 cited in the application
- WANG W ET AL: "A potato cDNA encoding a homologue of mammalian multidrug resistant P-glycoprotein." PLANT MOLECULAR BIOLOGY, vol. 31, no. 3, 1996, pages 683-687, XP002168734 ISSN: 0167-4412 cited in the application
- FLEENOR D ET AL: "NUCLEOTIDE SEQUENCE OF THE MAIZE MUTATOR ELEMENT MU8" NUCLEIC ACIDS RESEARCH, vol. 18, no. 22, 1990, page 6725 XP002168759 ISSN: 0305-1048
- BENNETZEN J L ET AL: "MOLECULAR CLONING OF MAIZE GENES BY TRANSPOSON TAGGING WITH MUTATOR" UCLA (UNIVERSITY OF CALIFORNIA LOS ANGELES) SYMPOSIA ON MOLECULAR AND, 1987, pages 183-204, XP001004827 1987 ISSN: 0735-9543
- SIDLER MICHAEL ET AL: "Involvement of an ABC transporter in a developmental pathway regulating hypocotyl cell elongation in the light." PLANT CELL, vol. 10, no. 10, October 1998 (1998-10), pages 1623-1636, XP002925142 ISSN: 1040-4651
- CAMPBELL L G ET AL: "EFFECTS OF A SINGLE HEIGHT GENE DW-3 OF SORGHUM ON CERTAIN AGRONOMIC CHARACTERS" CROP SCIENCE, vol. 15, no. 4, 1975, pages 595-597, XP001006317 ISSN: 0011-183X
- SPRAY CLIVE R ET AL: "Cloning a maize dwarfing gene by transposon tagging." PLANT PHYSIOLOGY (ROCKVILLE), vol. 108, no. 2 SUPPL., 1995, page 132 XP002168736 Annual Meeting of the American Society of Plant Physiologists;Charlotte, North Carolina, USA; July 29-August 2, 1995 ISSN: 0032-0889
- DAVIES T G EMYR ET AL: "Cloning and characterisation of a novel P-glycoprotein homologue from barley." GENE (AMSTERDAM), vol. 199, no. 1-2, 1997, pages 195-202, XP002925141 ISSN: 0378-1119

### SEQUENCE LISTING

<110> Johal, Gurmukh S
   Multani, Dilbag S
<120> SORGHUM DWARFING GENES AND METHODS OF USE
<130> 5718-100 (035718/205458)
<140>
   <141>
<150> 60/165,176
   <151> 1999-11-12
<160> 9
<170> PatentIn Ver. 2.1
<210> 1
   <211> 2139
   <212> DNA
   <213> Sorghum bicolor
<400> 1
   ctcctcgccg tgttcccgct cgtcgtgggc gccaccgtgc tgcagaagat gttcatgaag 60
   ggcttctcgg gggacctgga ggccgcgcac gccagggcca cgcagatcgc gggcgaggcc 120
   gtggccaacc tgcgcaccgt ggccgcgttc aacgcggagc gcaagatcac ggggctgttc 180
   gaggccaacc tgcgcggccc gctccggcgc tgcttctgga aggggcagat cgccggcagc 240
   ggctacggcg tggcgcagtt cctgctgtac gcgtcctacg cgctggggct gtggtacgcg 300
   gcgtggctgg tgaagcacgg cgtgtccgac ttctcgcgca ccatccgcgt gttcatggtg 360
   ctgatggtgt ccgccaacgg cgccgccgag acgctgacgc tggcgccgga ctttgtcaag 420
   ggcgggcgcg cgatgcggtc cgtgttcgag accatcgacc ggaaaacgga ggtggagccc 480
   gacgacgtgg acgcggcgcc ggtgccggag cggcccaagg gcgaggtgga gctgaagcac 540
   gtggacttct cgtacccgtc gcggccggac atccaggtgt tccgcgacct gagcctccgg 600
   gcgcgcgccg ggaagacgct ggcgctggtg ggtccgagcg ggtgcggcaa gagctcggtg 660
   ctggcgctgg cgcagcggtt ctacgagccc acgtccgggc gcgtgctcct ggacggcaag 720
   gacgtgcgca agtacaacct gcgggcgctg cggcgcgtgg tggcggtggt gccgcaggag 780
   ccgttcctgt tcgcggcgag catccacgac aacatcgcgt acgggcgcga gggcgcgacg 840
   gaggcggagg tggtggaggc ggcgactcaa gcgaacgcgc accggttcat ctcggcgctg 900
   ccggagggct acgggacgca agtgggcgag cgcggggtgc agctgtcggg cggcagcggg 960
   cagcggatcg cgatcgcgcg cgcgctggtg aagcaagcgg ccatcatgcc gctggacgag 1020
   gcgaccagcg cgctggaccc gagtcggagc ggtggctctt cgaggccaac cttcgcggcc 1080
   cgctccggcg cttgttctgg aaggggcaga tcgcgggaac ggtacgggcg tggcgcagtt 1140
   cttgctgacg cgtcctacgc gcttggggtt ttggtacccc gcgtggctag tgaagcacgg 1200
   gtctccgact ttcgcgcacc atccgggtgt tcatggtgct gatggtgtcc gccaacggcg 1260
   ccgccgagac gctgacgctg gcgccggact ttgtcaaggg cgggcgcgcg atgcggtccg 1320
   tgttcgagac catcgaccgg aaaacggagg tggagcccga cgacgtggac gcggcgccgg 1380
   tgccggagcg gcccaagggc gaggtggagc tgaagcacgt ggacttctcg tacccgtcgc 1440
   ggccggacat ccaggtgttc cgcgacctga gcctccgggc gcgcgccggg aagacgctgg 1500
   cgctggtggg tccgagcggg tgcggcaaga gctcggtgct ggcgctggtg cagcggttct 1560
   acgagcccac gtccgggcgc gtgctcctgg acggcaagga cgtgcgcaag tacaacctgc 1620
   gggcgctgcg gcgcgtggtg gcggtggtgc cgcaggagcc gttcctgttc gcggcgagca 1680
   tccacgacaa catcgcgtac gggcgcgagg gcgcgacgga ggcggaggtg gtggaggcgg 1740
   cgacgcaggc gaacgcgcac cggttcatct cggcgctgcc ggagggctac gggacgcagg 1800
   tgggcgagcg cggggtgcag ctgtcgggcg ggcagcggca gcggatcgcg atcgcgcgcg 1860
   ctggtaagca gcggccatcg tgctgctgga cgaggcgacc agcgcgctgg acgccgagtc 1920
   ggagcggtgc gtgcaggagg cgctggagcg cgcggggtcc gggcgcacca ccatcgtggt 1980
   ggcgcaccgg ctggccacgg tgcgcggcgc gcacaccatc gcggtcatcg acgacggcaa 2040
   ggtggcggag caggggtcgc actcgcacct gctcaagcac catcccgacg ggtgctacgc 2100
   gcggatgctg cagctgcagc ggctgacggg cgcggcggc                        2139
<210> 2
   <211> 1267
   <212> DNA
   <213> Sorghum bicolor
<400> 2
   ctcctcgccg tgttcccgct cgtcgtgggc gccaccgtgc tgcagaacat gttcatgaag 60
   ggcttctcgg gggacctgga ggccgcgcac gccagggcca cgcagatcgc gggcgaggcc 120
   gtggccaacc tgcgcaccgt ggccgcgttc aacgcggagc gcaagatcac ggggctgttc 180
   gaggccaacc tgcgcggccc gctccggcgc tgcttctgga aggggcagat cgccggcagc 240
   ggctacggcg tggcgcagtt cctgctgtac gcgtcctacg cgctggggct gtggtacgcg 300
   gcgtggctgg tgaagcacgg cgtgtccgac ttctcgcgca ccatccgcgt gttcatggtg 360
   ctgatggtgt ccgcgaacgg gcgcccgccg agacgctgac gctggcgccg gacttcatca 420
   agggcgggcg cgcgatgcgg tcggtgttcg agacgatcga ccgcaagacg gaggtggagc 480
   ccgacgacgt ggacgcggcg ccggtgccgg agcggccgag gggcgaggtg gagctgaagc 540
   acgtggactt ctcgtacccg tcgcggccgg acatccaggt gttccgcgac ctgagcctcc 600
   gtgcgcgcgc cgggaagacg ctggcgctgg tggggccgag cgggtgcggc aagagctcgg 660
   tgctggctct ggtgcagcgg ttctacaagc ccacgtccgg gcgcgtgttc ttgacggcaa 720
   agacgtgcgc aaaaacaacc ttcgggcgtt ccggcgcatt gttgcggtgg tacccaagaa 780
   cccgtttcct gttcgcggcg aagaatccac gagaacatcg cgcacgggcg agagggcgct 840
   acggaggcgg aggtggtgga ggcggcggcg caggcgaacg cgcaccggtt catcgcggcg 900
   ctgccggaag ggtactggac cgcagaaggg cgagcgcggg gtgcacctgt cggggggcag 960
   cggcagcgga tcgcgatcgc gcgcgcgctg gtgaagcagc ggccatcgtg ctgctggacg 1020
   aggcgaccag cgcgctggac gccgagtcgg agcggtgcgt gcaggaggcg ctggagcgcg 1080
   cggggtccgg gcgcaccacc atcgtggtgg cgcaccggct ggccacggtg cgcggcgcgc 1140
   acaccatcgc ggtcatcgac gacggcaagg tggcggagca ggggtcgcac tcgcacctgc 1200
   tcaagcacca tcccgacggg tgctacgcgc ggatgctgca gctgcagcgg ctgacggcgg 1260
   cggcggc 1267
<210> 3
   <211> 1261
   <212> DNA
   <213> Sorghum bicolor
<220>
   <221> CDS
   <222> (1)..(1245)
<400> 3
   ctc ctc gcc gtg ttc ccg ctc gtc gtg ggc gcc acc gtg ctg cag aag 48
   Leu Leu Ala Val Phe Pro Leu Val Val Gly Ala Thr Val Leu Gln Lys
   1 5 10 15
   atg ttc atg aag ggc ttc tcg ggg gac ctg gag gcc gcg cac gcc agg 96
   Met Phe Met Lys Gly Phe Ser Gly Asp Leu Glu Ala Ala His Ala Arg
   20 25 30
   gcc acg cag atc gcg ggc gag gcc gtg gcc aac ctg cgc acc gtg gcc 144
   Ala Thr Gln Ile Ala Gly Glu Ala Val Ala Asn Leu Arg Thr Val Ala
   35 40 45
   gcg ttc aac gcg gag cgc aag atc acg ggg ctg ttc gag gcc aac ctg 192
   Ala Phe Asn Ala Glu Arg Lys Ile Thr Gly Leu Phe Glu Ala Asn Leu
   50 55 60
   cgc ggc ccg ctc cgg cgc tgc ttc tgg aag ggg cag atc gcc ggc agc 240
   Arg Gly Pro Leu Arg Arg Cys Phe Trp Lys Gly Gln Ile Ala Gly Ser
   65 70 75 80
   ggc tac ggc gtg gcg cag ttc ctg ctg tac gcg tcc tac gcg ctg ggg    288
   Gly Tyr Gly Val Ala Gln Phe Leu Leu Tyr Ala Ser Tyr Ala Leu Gly
                    85                  90                  95
   ctg tgg tac gcg gcg tgg ctg gtg aag cac ggc gtg tcc gac ttc tcg    336
   Leu Trp Tyr Ala Ala Trp Leu Val Lys His Gly Val Ser Asp Phe Ser
               100                 105                 110
   cgc acc atc cgc gtg ttc atg gtg ctg atg gtg tcc gcc aac ggc gcc    384
   Arg Thr Ile Arg Val Phe Met Val Leu Met Val Ser Ala Asn Gly Ala
           115                 120                 125
   gcc gag acg ctg acg ctg gcg ccg gac ttt gtc aag ggc ggg cgc gcg    432
   Ala Glu Thr Leu Thr Leu Ala Pro Asp Phe Val Lys Gly Gly Arg Ala
       130                 135                 140
   atg cgg tcc gtg ttc gag acc atc gac cgg aaa acg gag gtg gag ccc    480
   Met Arg Ser Val Phe Glu Thr Ile Asp Arg Lys Thr Glu Val Glu Pro
   145                 150                 155                 160
   gac gac gtg gac gcg gcg ccg gtg ccg gag cgg ccc aag ggc gag gtg    528
   Asp Asp Val Asp Ala Ala Pro Val Pro Glu Arg Pro Lys Gly Glu Val
                   165                 170                 175
   gag ctg aag cac gtg gac ttc tcg tac ccg tcg cgg ccg gac atc cag    576
   Glu Leu Lys His Val Asp Phe Ser Tyr Pro Ser Arg Pro Asp Ile Gln
               180                 185                 190
   gtg ttc cgc gac ctg agc ctc cgg gcg cgc gcc ggg aag acg ctg gcg    624
   Val Phe Arg Asp Leu Ser Leu Arg Ala Arg Ala Gly Lys Thr Leu Ala
           195                 200                 205
   ctg gtg ggt ccg agc ggg tgc ggc aag agc tcg gtg ctg gcg ctg gtg    672
   Leu Val Gly Pro Ser Gly Cys Gly Lys Ser Ser Val Leu Ala Leu Val
       210                 215                 220
   cag cgg ttc tac gag ccc acg tcc ggg cgc gtg ctc ctg gac ggc aag    720
   Gln Arg Phe Tyr Glu Pro Thr Ser Gly Arg Val Leu Leu Asp Gly Lys
   225                 230                 235                 240
   gac gtg cgc aag tac aac ctg cgg gcg ctg cgg cgc gtg gtg gcg gtg    768
   Asp Val Arg Lys Tyr Asn Leu Arg Ala Leu Arg Arg Val Val Ala Val
                   245                 250                 255
   gcg ccg cag gag ccg ttc ctg ttc gcg gcg agc atc cac gac aac atc    816
   Ala Pro Gln Glu Pro Phe Leu Phe Ala Ala Ser Ile His Asp Asn Ile
               260                 265                 270
   gcg tac ggg cgc gag ggc gcg acg gag gcg gag gtg gtg gag gcg gcg    864
   Ala Tyr Gly Arg Glu Gly Ala Thr Glu Ala Glu Val Val Glu Ala Ala
           275                 280                 285
   acg cag gcg aac gcg cac cgg ttc atc gcg gcg ctg ccg gag ggc tac    912
   Thr Gln Ala Asn Ala His Arg Phe Ile Ala Ala Leu Pro Glu Gly Tyr
       290                 295                 300
   ggg acg cag gtg ggc gag cgc ggg gtg cag ctg tcg ggc ggg cag cgg    960
   Gly Thr Gln Val Gly Glu Arg Gly Val Gln Leu Ser Gly Gly Gln Arg
   305                 310                 315                 320
   cag cgg atc gcg atc gcg cgc gct ggt aag cag cgg cca tcg tgc tgc    1008
   Gln Arg Ile Ala Ile Ala Arg Ala Gly Lys Gln Arg Pro Ser Cys Cys
                   325                 330                 335
   tgg acg agg cga cca gcg cgc tgg acg ccg agt cgg agc ggt gcg tgc    1056
   Trp Thr Arg Arg Pro Ala Arg Trp Thr Pro Ser Arg Ser Gly Ala Cys
               340                 345                 350
   agg agg cgc tgg agc gcg cgg ggt ccg ggc gca cca cca tcg tgg tgg    1104
   Arg Arg Arg Trp Ser Ala Arg Gly Pro Gly Ala Pro Pro Ser Trp Trp
           355                 360                 365
   cgc acc ggc tgg cca cgg tgc gcg gcg cgc aca cca tcg cgg tca tcg    1152
   Arg Thr Gly Trp Pro Arg Cys Ala Ala Arg Thr Pro Ser Arg Ser Ser
       370                 375                 380
   acg acg gca agg tgg cgg agc agg ggt cgc act cgc acc tgc tca agc    1200
   Thr Thr Ala Arg Trp Arg Ser Arg Gly Arg Thr Arg Thr Cys Ser Ser
   385                 390                 395                 400
   acc atc ccg acg ggt gct acg cgc gga tgc tgc agc tgc agc ggc        1245
   Thr Ile Pro Thr Gly Ala Thr Arg Gly Cys Cys Ser Cys Ser Gly
                   405                 410                 415
   tgacgggcgc ggcggc                                                  1261
<210> 4
   <211> 415
   <212> PRT
   <213> Sorghum bicolor
<400> 4
   Leu Leu Ala Val Phe Pro Leu Val Val Gly Ala Thr Val Leu Gln Lys
   1               5                   10                  15
   Met Phe Met Lys Gly Phe Ser Gly Asp Leu Glu Ala Ala His Ala Arg
               20                  25                  30
   Ala Thr Gln Ile Ala Gly Glu Ala Val Ala Asn Leu Arg Thr Val Ala
           35                  40                  45
   Ala Phe Asn Ala Glu Arg Lys Ile Thr Gly Leu Phe Glu Ala Asn Leu
       50                  55                  60
   Arg Gly Pro Leu Arg Arg Cys Phe Trp Lys Gly Gln Ile Ala Gly Ser
   65                  70                  75                  80
   Gly Tyr Gly Val Ala Gln Phe Leu Leu Tyr Ala Ser Tyr Ala Leu Gly
                   85                  90                  95
   Leu Trp Tyr Ala Ala Trp Leu Val Lys His Gly Val Ser Asp Phe Ser
               100                 105                 110
   Arg Thr Ile Arg Val Phe Met Val Leu Met Val Ser Ala Asn Gly Ala
           115                 120                 125
   Ala Glu Thr Leu Thr Leu Ala Pro Asp Phe Val Lys Gly Gly Arg Ala
       130                 135                 140
   Met Arg Ser Val Phe Glu Thr Ile Asp Arg Lys Thr Glu Val Glu Pro
   145                 150                 155                 160
   Asp Asp Val Asp Ala Ala Pro Val Pro Glu Arg Pro Lys Gly Glu Val
                   165                 170                 175
   Glu Leu Lys His Val Asp Phe Ser Tyr Pro Ser Arg Pro Asp Ile Gln
               180                 185                 190
   Val Phe Arg Asp Leu Ser Leu Arg Ala Arg Ala Gly Lys Thr Leu Ala
           195                 200                 205
   Leu Val Gly Pro Ser Gly Cys Gly Lys Ser Ser Val Leu Ala Leu Val
       210                 215                 220
   Gln Arg Phe Tyr Glu Pro Thr Ser Gly Arg Val Leu Leu Asp Gly Lys
   225                 230                 235                 240
   Asp Val Arg Lys Tyr Asn Leu Arg Ala Leu Arg Arg Val Val Ala Val
                   245                 250                 255
   Ala Pro Gln Glu Pro Phe Leu Phe Ala Ala Ser Ile His Asp Asn Ile
               260                 265                 270
   Ala Tyr Gly Arg Glu Gly Ala Thr Glu Ala Glu Val Val Glu Ala Ala
           275                 280                 285
   Thr Gln Ala Asn Ala His Arg Phe Ile Ala Ala Leu Pro Glu Gly Tyr
       290                 295                 300
   Gly Thr Gln Val Gly Glu Arg Gly Val Gln Leu Ser Gly Gly Gln Arg
   305                 310                 315                 320
   Gln Arg Ile Ala Ile Ala Arg Ala Gly Lys Gln Arg Pro Ser Cys Cys
                   325                 330                 335
   Trp Thr Arg Arg Pro Ala Arg Trp Thr Pro Ser Arg Ser Gly Ala Cys
               340                 345                 350
   Arg Arg Arg Trp Ser Ala Arg Gly Pro Gly Ala Pro Pro Ser Trp Trp
           355                 360                 365
   Arg Thr Gly Trp Pro Arg Cys Ala Ala Arg Thr Pro Ser Arg Ser Ser
       370                 375                 380
   Thr Thr Ala Arg Trp Arg Ser Arg Gly Arg Thr Arg Thr Cys Ser Ser
   385                 390                 395                 400
   Thr Ile Pro Thr Gly Ala Thr Arg Gly Cys Cys Ser Cys Ser Gly
                   405                 410                 415
<210> 5
   <211> 26
   <212> DNA
   <213> Artificial Sequence
<220>
   <223> Description of Artificial Sequence:oligonucleotide primer designed from sequence of Zea mays Br2 gene
<400> 5
   ctcctcgccg tgttcccgct cgtcgt 26
<210> 6
   <211> 17
   <212> DNA
   <213> Artificial Sequence
<220>
   <223> Description of Artificial Sequence:oligonucleotide primer designed from sequence of Zea mays Br2 gene
<400> 6
   gccgccgcgc ccgtcag 17
<210> 7
   <211> 6827
   <212> DNA
   <213> Sorghum bicolor
<400> 7
   gtccctcccc ggcccccgat cgatgtctac caacgacccg gacgagatca gggcgcgcgt 60
   cgtcgtcctc ggcgcccctc atgccgacga cgacgccggc gacgagtggg cccgccccga 120
   gctcgaggcc ttccacctcc cctctcccgc ccaccagcct cctggcttcc acctagccgc 180
   tgggcaccaa ccggaagctg cagcagagca acccaccacg ctccctgctg cccgccgcac 240
   cagcgacaca tccactgctg ctggtgctgc tcctccttct ccttcgccgc ctccgccgcc 300
   ggctcctttg gagatggacc agccgcccaa tgccaagccg gcctcctcct ccgccgccgc 360
   cgccggcgcc aatgacaaca agaagcccac cccgcccgcc gcgctgcgcg acctcttccg 420
   cttcgccgac ggcctcgact gcgcgctcat gctcgtcggc acgctcggcg cgctcgtcca 480
   cggctgctcg ctccccgtct tcctccgctt cttcgccgac ctcgtcgact ccttcggctc 540
   ccacgccaac gacccggaca ccatggtccg cctcgtcgtc aagtacgcct tctacttcct 600
   cgtcgtcgga gccgcaatct gggcgtcctc atgggcaggt aaccaacgtt attcctcctc 660
   ctcctcccct ccctcctccc ggcactgctg ctcgcgtcgc gaattgtctg tcgatttgga 720
   ttggatggcg aatcacatca gtcgctcaat cttcatggcc catggctagc aatgagatcg 780
   accttcgaat ccctcgcttg cagagatctc ctgctggatg tggaccggcg agcggcagtc 840
   gacgcggatg cggatccggt acctggacgc ggcgctgcgg caggacgtgt ccttcttcga 900
   caccgacgtg cgcacctcgg acgtcatcta cgccatcaac gcggacgccg tggtgggtgc 960
   aggacgccat cagcgagaag ctgggcaacc tcatccacta catggccacc ttcgtggcgg 1020
   gcttcgtcgt gggcttcacc gccgcctggc agctggcgct cgtcacgctc gccgtcgtgc 1080
   cgctcatcgc cgtcatcggg gggctcagcg ccgccgcgct cgccaagctc tcctccagga 1140
   gccaggacgc gctgtcgggc gccagcggca tcgcggagca ggcgctcgcg cagatacgga 1200
   tcgtgcaggc cttcgtcggc gaggagcgcg aaatgcgggc gtactcggcg gcgttggccg 1260
   tcgcgcagaa gatcggctac cgcagcggct tcgccaaggg gctcggcctc ggcggcacct 1320
   acttcaccgt cttctgctgc tacggcctcc tgctctggta cggcggacac ctcgtccgcg 1380
   gaaccacacc aacggagggc tcgccatcgc caccatgttc tccgtcatga tcggcgggct 1440
   gtaagatgat cagtttctcc cgggctctcc tgttcttccg tcatgacaca gcatgtacta 1500
   cgtacgctta ctggtctgtg tctgtgtgtg tgtggatcgc ctgcgtccag ggccctcggg 1560
   cagtcggcgc cgagcatggc cgcgttcgcc aaggcgcgcg tggcggccgc caagatcttc 1620
   cgcatcatcg accacaggcc gggcatctcc tcgcgggacg gcgaggacgg cggcggcgtg 1680
   gagctggagt cggtgacggg gcgggtggag atgaggggcg tggacttcgc gtacccgtcg 1740
   cggccggacg tccccatcct gcgcggcttc tcgctcagcg tgcccgccgg caagaccatc 1800
   gcgctggtgg gcagctccgg ctccgggaag agcacggtgg tgtcgctcct cgagaggttc 1860
   tacgacccca gcgcaggtat acatagtacg ctaccaattc tagctttagc gcattgatta 1920
   attagtgttg gagttcactt gcttgccaat tgccattgcc atcacacatc agcagctacc 1980
   atacattgcc aactgccatt gctgctgcct tgctgggtgg ttagtagggg aagaagcttc 2040
   cactgtagca ggagtacatt gcaaacagga agtgaatttt gcacgtggga aatgaagaag 2100
   tgaatgcttg gagcagagct ggccggcctc atgggctgct tacctactat ctagtcaacc 2160
   aagcatccct gtttcttcct tgtttatggt caaggcattc acaccagctt agaaacttag 2220
   aaagaagcta agccctttgt tttaattttc ctaaaaaaaa atttttggtt aaaatttttt 2280
   taaagtttcc cttgtccaca atcccaaatt cttttaagaa gtattaatat atgacgaaaa 2340
   ttaaaaccta attgcaccag ttttggtcga aattgaccga aaacgatctt ttgagcctaa 2400
   ttagtcccat gattggacaa tatttgtcaa atacaaacga aagtgctgcc gtatcgattt 2460
   tgcaaaggtt ttcggaacta aacaaagcct ggtgcctgca acgcgagaca aagaaaacta 2520
   tttgcctggc aagatgccac tattgcacat gcatgccact ctttgagcct tgaccgactg 2580
   actgactact cagagtagga gtggttcaat tgtattgaca tgtagtagga gtactcgtat 2640
   gctatagtag tcctgtagct ttttcaaaca aaaaaaaaag agaaagaaag aaatgaagtc 2700
   tgaaatttgt tggttttggc agggcaaatc ttgctggacg ggcatgatct caagtcgctg 2760
   aagctccggt ggctccggca gcagattggt ctggtgagcc aggagccgac gctgttcgcg 2820
   acgagcatca aggagaacct gctgctgggg cgggacagtc agagtgcgac gcaggccgag 2880
   atggaggagg ccgccagggt ggccaacgcg cactccttca tcgtcaagct ccccgacggc 2940
   tacgacacgc aggtccgtcc cgtatagcta gctcactagc tgcactgcca cttctctcgc 3000
   ttgctcccca ccgttgctgc ctgttgctct ccaatccact tgtcggtgtc tggaccacac 3060
   gtgcctgctt gcctagctgc tccacatctg ctttccctgt ccaaccttat gcaactcact 3120
   ctaatactat atcaaataca tttctagagt ttaaagctta tcttagaata aatgcatctt 3180
   tagctacgag acaacctaac ttcagttgtt gttgttgttt tttttacttt ctctcttctc 3240
   acaaatacta tgattacgtc tttacagcga tcttttttat tccaaaccta aaaatgcatg 3300
   cactcactct aaaagcgcaa agggagcatc tttttttccc ccatcatctg cacgcagcct 3360
   tttcttttcc tcatgtcacg agggactgaa ggtgtgtatg cagcgtcaag tcatccatcc 3420
   gttccacttg gattaggacg ggggggccaa tttttaggcc ccttgttgcc attcgcattt 3480
   tgttgttgtg ggtttgccaa caagaaactt gccaggttgc ttttgttatc acgcacagga 3540
   caggagaggt ctttttctcg acacaagctc tacagcctct actaaactag cacttgctga 3600
   tgagagcaga ggatgaatgg acgatgaaca tctagagtga gagagaaaaa aatgttaata 3660
   ataataaaaa gtagtagcag gattaagaat caacctgggg tacgtaggaa gaggtacaat 3720
   ccctaggaat ctagagtatg agaagtatgg gaggagttgg gggagtggaa cggaacaaat 3780
   tccgagttgg tattttgccg ggaatgtcaa gttgattttt gatcctagtg caagcaagaa 3840
   ttatcaatca ctcagactca gcctgtctgt gtctgtccac cccagctctt gctactctac 3900
   ttactactgt gctactagtg gtagggtagg tatcttacat aaactgttat tataaactgt 3960
   catctgagaa agagagccag tcaaacccat gctgctgctt attttaatca ctgtcaaatg 4020
   gcaggcaggc aggcagtctg gttagttaat aacatctggg aagggtttaa tcaaaccaaa 4080
   tcaaatcaga cgaaatctag aggccacatg ggatggggcc atatgtactg tactagcata 4140
   actagcggct agattttatt agaacacgga ctcacactcc cataactata actgacttga 4200
   tcatgattcc ttgccaagca atgctcgcat gcccatgcat gcatcatccc tggtcaaact 4260
   caaacactct ccaccgtcag ggaataagac ttattatttt attaacaatt caatttttat 4320
   ttattaatta cgtctggacg aggagtactg gtttatttga tgagagacat ggcagtccaa 4380
   gtcaaactcg tttgtctgac catggcggtg atggccggtg caggttgggg agcgcggcct 4440
   gcagctctcc ggtgggcaga agcagcgcat cgccatcgcc cgcgccatgc tcaagaaccc 4500
   cgccatcctg ctgctggacg aggccaccag cgcgctggac tccgagtctg agaagctcgt 4560
   gcaggaggcg ctggaccgct tcatgatcgg gcgcaccacc ctggtgatcg cgcacaggat 4620
   gtccaccatc cgcaaggccg acgtggtggc cgtgctgcag ggcggccccg tctccgagat 4680
   gggcgcgcac gacgagttga tggccaaggg cgagaacggc acttacgcca agttcatccg 4740
   catgcaggag caggcgcacg aggcggcgtt cgtcaacgcc cgccgcagca gcgccaggcc 4800
   ctccagcgcc cgcaactccg tcagctcgcc catcatgacg cgcaactcct cctacggccg 4860
   ctccccatac tcccgccgcc tctccgactt ctccacctcc gacttcaccc tctccatcca 4920
   cgacccgcac caccaccacc ggacgatggc cgacaagcag ctcgcgttcc gcgccggcgc 4980
   cagctccttc ctccgcctcg ccaggatgaa ctcgcccgag tgggcctacg cgctcgtcgg 5040
   ctccctgggc tccatggtct gcggctcctt cagcgccatc ttcgcctaca tcctcagcgc 5100
   cgtgctcagc gtctactacg cgccggaccc tcgctacatg aagcgcgaga tcgccaagta 5160
   ctgctacctg ctcatcggca tgtcctccgc ggcgctgctg ttcaacacgg tgcagcacgt 5220
   gttctgggac acggtcggcg agaacctcac gaagcgtgtg cgcgagaaga tgttcgccgc 5280
   cgtgctccgc aacgagatcg cctggttcga cgccgacgag aacgccagcg cgcgcgtcgc 5340
   cgccaggctc gcgctcgacg cccagaacgt gcgctccgcc atcggggacc gtatctccgt 5400
   catcgtccag aactcggcgc tcatgctcgt cgcctgcacc gcgggcttcg tcctccagtg 5460
   gcgcctcgcg ctcgtgctcc tcgccgtctt cccgctcgtc gtggccgcca ccgtgctgca 5520
   gaagatgttc atgaagggct tctcggggga cctggaggcc gcgcacgcca gggccacgca 5580
   gatcgcgggc gaggccgtgg ccaacctgcg caccgtggcc gcgttcaacg cggagcgcaa 5640
   gatcacgggg ctgttcgagg ccaacctgcg cggcccgctc cggcgctgct tctggaaggg 5700
   gcagatcgcc ggcagcggct acggcgtggc gcagttcctg ctgtacgcgt cctacgcgct 5760
   ggggctgtgg tacgcggcgt ggctggtgaa gcacggcgtg tccgacttct cgcgcaccat 5820
   ccgcgtgttc atggtgctga tggtgtccgc caacggcgcc gccgagacgc tgacgctggc 5880
   gccggacttt gtcaagggcg ggcgcgcgat gcggtccgtg ttcgagacca tcgaccggaa 5940
   aacggaggtg gagcccgacg acgtggacgc ggcgccggtg ccggagcggc ccaagggcga 6000
   ggtggagctg aagcacgtgg acttctcgta cccgtcgcgg ccggacatcc aggtgttccg 6060
   cgacctgagc ctccgggcgc gcgccgggaa gacgctggcg ctggtgggtc cgagcgggtg 6120
   cggcaagagc tcggtgctgg cgctggtgca gcggttctac gagcccacgt ccgggcgcgt 6180
   gctcctggac ggcaaggacg tgcgcaagta caacctgcgg gcgctgcggc gcgtggtggc 6240
   ggtggcgccg caggagccgt tcctgttcgc ggcgagcatc cacgacaaca tcgcgtacgg 6300
   gcgcgagggc gcgacggagg cggaggtggt ggaggcggcg acgcaggcga acgcgcaccg 6360
   gttcatcgcg gcgctgccgg agggctacgg gacgcaggtg ggcgagcgcg gggtgcagct 6420
   gtcgggcggg cagcggcagc ggatcgcgat cgcgcgcgcg ctggtgaagc aggcggccat 6480
   cgtgctgctg gacgaggcga ccagcgcgct ggacgccgag tcggagcggt gcgtgcagga 6540
   ggcgctggag cgcgcggggt ccgggcgcac caccatcgtg gtggcgcacc ggctggccac 6600
   ggtgcgcggc gcgcacacca tcgcggtcat cgacgacggc aaggtggcgg agcaggggtc 6660
   gcactcgcac ctgctcaagc accatcccga cgggtgctac gcgcggatgc tgcagctgca 6720
   gcggctgacg ggcgggtgcc gcgcccgggc cgccgccgtc gtcgtccaac ggggccgccg 6780
   cgtaggatgg atggatggat catggatgag tttggttcct tgataaa              6827
<210> 8
   <211> 4213
   <212> DNA
   <213> Sorghum bicolor
<220>
   <221> CDS
   <222> (1)..(4206)
<400> 8
   atg tct acc aac gac ccg gac gag atc agg gcg cgc gtc gtc gtc ctc    48
   Met Ser Thr Asn Asp Pro Asp Glu Ile Arg Ala Arg Val Val Val Leu
    1               5                  10                  15
   ggc gcc cct cat gcc gac gac gac gcc ggc gac gag tgg gcc cgc ccc    96
   Gly Ala Pro His Ala Asp Asp Asp Ala Gly Asp Glu Trp Ala Arg Pro
               20                  25                  30
   gag ctc gag gcc ttc cac ctc ccc tct ccc gcc cac cag cct cct ggc   144
   Glu Leu Glu Ala Phe His Leu Pro Ser Pro Ala His Gln Pro Pro Gly
           35                  40                  45
   ttc cac cta gcc gct ggg cac caa ccg gaa gct gca gca gag caa ccc   192
   Phe His Leu Ala Ala Gly His Gln Pro Glu Ala Ala Ala Glu Gln Pro
       50                  55                  60
   acc acg ctc cct gct gcc cgc cgc acc agc gac aca tcc act gct gct   240
   Thr Thr Leu Pro Ala Ala Arg Arg Thr Ser Asp Thr Ser Thr Ala Ala
   65                  70                  75                  80
   ggt gct gct cct cct tct cct tcg ccg cct ccg ccg ccg gct cct ttg   288
   Gly Ala Ala Pro Pro Ser Pro Ser Pro Pro Pro Pro Pro Ala Pro Leu
                   85                  90                  95
   gag atg gac cag ccg ccc aat gcc aag ccg gcc tcc tcc tcc gcc gcc   336
   Glu Met Asp Gln Pro Pro Asn Ala Lys Pro Ala Ser Ser Ser Ala Ala
               100                 105                 110
   gcc gcc ggc gcc aat gac aac aag aag ccc acc ccg ccc gcc gcg ctg   384
   Ala Ala Gly Ala Asn Asp Asn Lys Lys Pro Thr Pro Pro Ala Ala Leu
           115                 120                 125
   cgc gac ctc ttc cgc ttc gcc gac ggc ctc gac tgc gcg ctc atg ctc   432
   Arg Asp Leu Phe Arg Phe Ala Asp Gly Leu Asp Cys Ala Leu Met Leu
           130                 135                 140
   gtc ggc acg ctc ggc gcg ctc gtc cac ggc tgc tcg ctc ccc gtc ttc      480
   Val Gly Thr Leu Gly Ala Leu Val His Gly Cys Ser Leu Pro Val Phe
   145                 150                 155                 160
   ctc cgc ttc ttc gcc gac ctc gtc gac tcc ttc ggc tcc cac gcc aac      528
   Leu Arg Phe Phe Ala Asp Leu Val Asp Ser Phe Gly Ser His Ala Asn
                   165                 170                 175
   gac ccg gac acc atg gtc cgc ctc gtc gtc aag tac gcc ttc tac ttc      576
   Asp Pro Asp Thr Met Val Arg Leu Val Val Lys Tyr Ala Phe Tyr Phe
               180                 185                 190
   ctc gtc gtc gga gcc gca atc tgg gcg tcc tca tgg gca gag atc tcc      624
   Leu Val Val Gly Ala Ala Ile Trp Ala Ser Ser Trp Ala Glu Ile Ser
           195                 200                 205
   tgc tgg atg tgg acc ggc gag cgg cag tcg acg cgg atg cgg atc cgg      672
   Cys Trp Met Trp Thr Gly Glu Arg Gln Ser Thr Arg Met Arg Ile Arg
       210                 215                 220
   tac ctg gac gcg gcg ctg cgg cag gac gtg tcc ttc ttc gac acc gac      720
   Tyr Leu Asp Ala Ala Leu Arg Gln Asp Val Ser Phe Phe Asp Thr Asp
   225                 230                 235                 240
   gtg cgc acc tcg gac gtc atc tac gcc atc aac gcg gac gcc gtg gtg      768
   Val Arg Thr Ser Asp Val Ile Tyr Ala Ile Asn Ala Asp Ala Val Val
                   245                 250                 255
   ggt gca gga cgc cat cag cga gaa gct ggg caa cct cat cca cta cat      816
   Gly Ala Gly Arg His Gln Arg Glu Ala Gly Gln Pro His Pro Leu His
               260                 265                 270
   ggc cac ctt cgt ggc ggg ctt cgt cgt ggg ctt cac cgc cgc ctg gca      864
   Gly His Leu Arg Gly Gly Leu Arg Arg Gly Leu His Arg Arg Leu Ala
           275                 280                 285
   gct ggc gct cgt cac gct cgc cgt cgt gcc gct cat cgc cgt cat cgg      912
   Ala Gly Ala Arg His Ala Arg Arg Arg Ala Ala His Arg Arg His Arg
       290                 295                 300
   ggg gct cag cgc cgc cgc gct cgc caa gct ctc ctc cag gag cca gga      960
   Gly Ala Gln Arg Arg Arg Ala Arg Gln Ala Leu Leu Gln Glu Pro Gly
   305                 310                 315                 320
   cgc gct gtc ggg cgc cag cgg cat cgc gga gca ggc gct cgc gca gat     1008
   Arg Ala Val Gly Arg Gln Arg His Arg Gly Ala Gly Ala Arg Ala Asp
                   325                 330                 335
   acg gat cgt gca ggc ctt cgt cgg cga gga gcg cga aat gcg ggc gta     1056
   Thr Asp Arg Ala Gly Leu Arg Arg Arg Gly Ala Arg Asn Ala Gly Val
               340                 345                 350
   ctc ggc ggc gtt ggc cgt cgc gca gaa gat cgg cta ccg cag cgg ctt     1104
   Leu Gly Gly Val Gly Arg Arg Ala Glu Asp Arg Leu Pro Gln Arg Leu
           355                 360                 365
   cgc caa ggg gct cgg cct cgg cgg cac cta ctt cac cgt ctt ctg ctg     1152
   Arg Gln Gly Ala Arg Pro Arg Arg His Leu Leu His Arg Leu Leu Leu
       370                 375                 380
   cta cgg cct cct gct ctg gta cgg cgg aca cct cgt ccg cgg aac cac     1200
   Leu Arg Pro Pro Ala Leu Val Arg Arg Thr Pro Arg Pro Arg Asn His
   385                 390                 395                 400
   acc aac gga ggg ctc gcc atc gcc acc atg ttc tcc gtc atg atc ggc     1248
   Thr Asn Gly Gly Leu Ala Ile Ala Thr Met Phe Ser Val Met Ile Gly
                   405                 410                 415
   ggg ctg gcc ctc ggg cag tcg gcg ccg agc atg gcc gcg ttc gcc aag     1296
   Gly Leu Ala Leu Gly Gln Ser Ala Pro Ser Met Ala Ala Phe Ala Lys
               420                 425                 430
   gcg cgc gtg gcg gcc gcc aag atc ttc cgc atc atc gac cac agg ccg     1344
   Ala Arg Val Ala Ala Ala Lys Ile Phe Arg Ile Ile Asp His Arg Pro
           435                 440                 445
   ggc atc tcc tcg cgg gac ggc gag gac ggc ggc ggc gtg gag ctg gag     1392
   Gly Ile Ser Ser Arg Asp Gly Glu Asp Gly Gly Gly Val Glu Leu Glu
       450                 455                 460
   tcg gtg acg ggg cgg gtg gag atg agg ggc gtg gac ttc gcg tac ccg     1440
   Ser Val Thr Gly Arg Val Glu Met Arg Gly Val Asp Phe Ala Tyr Pro
   465                 470                 475                 480
   tcg cgg ccg gac gtc ccc atc ctg cgc ggc ttc tcg ctc agc gtg ccc     1488
   Ser Arg Pro Asp Val Pro Ile Leu Arg Gly Phe Ser Leu Ser Val Pro
                   485                 490                 495
   gcc ggc aag acc atc gcg ctg gtg ggc agc tcc ggc tcc ggg aag agc     1536
   Ala Gly Lys Thr Ile Ala Leu Val Gly Ser Ser Gly Ser Gly Lys Ser
               500                 505                 510
   acg gtg gtg tcg ctc ctc gag agg ttc tac gac ccc agc gca ggg caa     1584
   Thr Val Val Ser Leu Leu Glu Arg Phe Tyr Asp Pro Ser Ala Gly Gln
           515                 520                 525
   atc ttg ctg gac ggg cat gat ctc aag tcg ctg aag ctc cgg tgg ctc     1632
   Ile Leu Leu Asp Gly His Asp Leu Lys Ser Leu Lys Leu Arg Trp Leu
       530                 535                 540
   cgg cag cag att ggt ctg gtg agc cag gag ccg acg ctg ttc gcg acg     1680
   Arg Gln Gln Ile Gly Leu Val Ser Gln Glu Pro Thr Leu Phe Ala Thr
   545                 550                 555                 560
   agc atc aag gag aac ctg ctg ctg ggg cgg gac agt cag agt gcg acg     1728
   Ser Ile Lys Glu Asn Leu Leu Leu Gly Arg Asp Ser Gln Ser Ala Thr
                   565                 570                 575
   cag gcc gag atg gag gag gcc gcc agg gtg gcc aac gcg cac tcc ttc     1776
   Gln Ala Glu Met Glu Glu Ala Ala Arg Val Ala Asn Ala His Ser Phe
               580                 585                 590
   atc gtc aag ctc ccc gac ggc tac gac acg cag gtt ggg gag cgc ggc     1824
   Ile Val Lys Leu Pro Asp Gly Tyr Asp Thr Gln Val Gly Glu Arg Gly
           595                 600                 605
   ctg cag ctc tcc ggt ggg cag aag cag cgc atc gcc atc gcc cgc gcc     1872
   Leu Gln Leu Ser Gly Gly Gln Lys Gln Arg Ile Ala Ile Ala Arg Ala
       610                 615                 620
   atg ctc aag aac ccc gcc atc ctg ctg ctg gac gag gcc acc agc gcg    1920
   Met Leu Lys Asn Pro Ala Ile Leu Leu Leu Asp Glu Ala Thr Ser Ala
   625                 630                 635                 640
   ctg gac tcc gag tct gag aag ctc gtg cag gag gcg ctg gac cgc ttc    1968
   Leu Asp Ser Glu Ser Glu Lys Leu Val Gln Glu Ala Leu Asp Arg Phe
                   645                 650                 655
   atg atc ggg cgc acc acc ctg gtg atc gcg cac agg atg tcc acc atc    2016
   Met Ile Gly Arg Thr Thr Leu Val Ile Ala His Arg Met Ser Thr Ile
               660                 665                 670
   cgc aag gcc gac gtg gtg gcc gtg ctg cag ggc ggc ccc gtc tcc gag    2064
   Arg Lys Ala Asp Val Val Ala Val Leu Gln Gly Gly Pro Val Ser Glu
           675                 680                 685
   atg ggc gcg cac gac gag ttg atg gcc aag ggc gag aac ggc act tac    2112
   Met Gly Ala His Asp Glu Leu Met Ala Lys Gly Glu Asn Gly Thr Tyr
       690                 695                 700
   gcc aag ttc atc cgc atg cag gag cag gcg cac gag gcg gcg ttc gtc    2160
   Ala Lys Phe Ile Arg Met Gln Glu Gln Ala His Glu Ala Ala Phe Val
   705                 710                 715                 720
   aac gcc cgc cgc agc agc gcc agg ccc tcc agc gcc cgc aac tcc gtc    2208
   Asn Ala Arg Arg Ser Ser Ala Arg Pro Ser Ser Ala Arg Asn Ser Val
                   725                 730                 735
   agc tcg ccc atc atg acg cgc aac tcc tcc tac ggc cgc tcc cca tac    2256
   Ser Ser Pro Ile Met Thr Arg Asn Ser Ser Tyr Gly Arg Ser Pro Tyr
               740                 745                 750
   tcc cgc cgc ctc tcc gac ttc tcc acc tcc gac ttc acc ctc tcc atc    2304
   Ser Arg Arg Leu Ser Asp Phe Ser Thr Ser Asp Phe Thr Leu Ser Ile
           755                 760                 765
   cac gac ccg cac cac cac cac cgg acg atg gcc gac aag cag ctc gcg    2352
   His Asp Pro His His His His Arg Thr Met Ala Asp Lys Gln Leu Ala
       770                 775                 780
   ttc cgc gcc ggc gcc agc tcc ttc ctc cgc ctc gcc agg atg aac tcg    2400
   Phe Arg Ala Gly Ala Ser Ser Phe Leu Arg Leu Ala Arg Met Asn Ser
   785                 790                 795                 800
   ccc gag tgg gcc tac gcg ctc gtc ggc tcc ctg ggc tcc atg gtc tgc    2448
   Pro Glu Trp Ala Tyr Ala Leu Val Gly Ser Leu Gly Ser Met Val Cys
                   805                 810                 815
   ggc tcc ttc agc gcc atc ttc gcc tac atc ctc agc gcc gtg ctc agc    2496
   Gly Ser Phe Ser Ala Ile Phe Ala Tyr Ile Leu Ser Ala Val Leu Ser
               820                 825                 830
   gtc tac tac gcg ccg gac cct cgc tac atg aag cgc gag atc gcc aag    2544
   Val Tyr Tyr Ala Pro Asp Pro Arg Tyr Met Lys Arg Glu Ile Ala Lys
           835                 840                 845
   tac tgc tac ctg ctc atc ggc atg tcc tcc gcg gcg ctg ctg ttc aac    2592
   Tyr Cys Tyr Leu Leu Ile Gly Met Ser Ser Ala Ala Leu Leu Phe Asn
       850                 855                 860
   acg gtg cag cac gtg ttc tgg gac acg gtc ggc gag aac ctc acg aag    2640
   Thr Val Gln His Val Phe Trp Asp Thr Val Gly Glu Asn Leu Thr Lys
   865                 870                 875                 880
   cgt gtg cgc gag aag atg ttc gcc gcc gtg ctc cgc aac gag atc gcc      2688
   Arg Val Arg Glu Lys Met Phe Ala Ala Val Leu Arg Asn Glu Ile Ala
                   885                 890                 895
   tgg ttc gac gcc gac gag aac gcc agc gcg cgc gtc gcc gcc agg ctc      2736
   Trp Phe Asp Ala Asp Glu Asn Ala Ser Ala Arg Val Ala Ala Arg Leu
               900                 905                 910
   gcg ctc gac gcc cag aac gtg cgc tcc gcc atc ggg gac cgt atc tcc      2784
   Ala Leu Asp Ala Gln Asn Val Arg Ser Ala Ile Gly Asp Arg Ile Ser
           915                 920                 925
   gtc atc gtc cag aac tcg gcg ctc atg ctc gtc gcc tgc acc gcg ggc      2832
   Val Ile Val Gln Asn Ser Ala Leu Met Leu Val Ala Cys Thr Ala Gly
       930                 935                 940
   ttc gtc ctc cag tgg cgc ctc gcg ctc gtg ctc ctc gcc gtc ttc ccg      2880
   Phe Val Leu Gln Trp Arg Leu Ala Leu Val Leu Leu Ala Val Phe Pro
   945                 950                 955                 960
   ctc gtc gtg gcc gcc acc gtg ctg cag aag atg ttc atg aag ggc ttc      2928
   Leu Val Val Ala Ala Thr Val Leu Gln Lys Met Phe Met Lys Gly Phe
                   965                 970                 975
   tcg ggg gac ctg gag gcc gcg cac gcc agg gcc acg cag atc gcg ggc      2976
   Ser Gly Asp Leu Glu Ala Ala His Ala Arg Ala Thr Gln Ile Ala Gly
               980                 985                 990
   gag gcc gtg gcc aac ctg cgc acc gtg gcc gcg ttc aac gcg gag cgc      3024
   Glu Ala Val Ala Asn Leu Arg Thr Val Ala Ala Phe Asn Ala Glu Arg
           995                 1000                1005
   aag atc acg ggg ctg ttc gag gcc aac ctg cgc ggc ccg ctc cgg cgc      3072
   Lys Ile Thr Gly Leu Phe Glu Ala Asn Leu Arg Gly Pro Leu Arg Arg
       1010                1015                1020
   tgc ttc tgg aag ggg cag atc gcc ggc agc ggc tac ggc gtg gcg cag      3120
   Cys Phe Trp Lys Gly Gln Ile Ala Gly Ser Gly Tyr Gly Val Ala Gln
   1025                1030                1035                1040
   ttc ctg ctg tac gcg tcc tac gcg ctg ggg ctg tgg tac gcg gcg tgg      3168
   Phe Leu Leu Tyr Ala Ser Tyr Ala Leu Gly Leu Trp Tyr Ala Ala Trp
                   1045                1050                1055
   ctg gtg aag cac ggc gtg tcc gac ttc tcg cgc acc atc cgc gtg ttc      3216
   Leu Val Lys His Gly Val Ser Asp Phe Ser Arg Thr Ile Arg Val Phe
               1060                1065                1070
   atg gtg ctg atg gtg tcc gcc aac ggc gcc gcc gag acg ctg acg ctg      3264
   Met Val Leu Met Val Ser Ala Asn Gly Ala Ala Glu Thr Leu Thr Leu
           1075                1080                1085
   gcg ccg gac ttt gtc aag ggc ggg cgc gcg atg cgg tcc gtg ttc gag      3312
   Ala Pro Asp Phe Val Lys Gly Gly Arg Ala Met Arg Ser Val Phe Glu
       1090                1095                1100
   acc atc gac cgg aaa acg gag gtg gag ccc gac gac gtg gac gcg gcg      3360
   Thr Ile Asp Arg Lys Thr Glu Val Glu Pro Asp Asp Val Asp Ala Ala
   1105                1110                1115                1120
   ccg gtg ccg gag cgg ccc aag ggc gag gtg gag ctg aag cac gtg gac     3408
   Pro Val Pro Glu Arg Pro Lys Gly Glu Val Glu Leu Lys His Val Asp
                   1125                1130                1135
   ttc tcg tac ccg tcg cgg ccg gac atc cag gtg ttc cgc gac ctg agc     3456
   Phe Ser Tyr Pro Ser Arg Pro Asp Ile Gln Val Phe Arg Asp Leu Ser
               1140                1145                1150
   ctc cgg gcg cgc gcc ggg aag acg ctg gcg ctg gtg ggt ccg agc ggg     3504
   Leu Arg Ala Arg Ala Gly Lys Thr Leu Ala Leu Val Gly Pro Ser Gly
           1155                1160                1165
   tgc ggc aag agc tcg gtg ctg gcg ctg gtg cag cgg ttc tac gag ccc     3552
   Cys Gly Lys Ser Ser Val Leu Ala Leu Val Gln Arg Phe Tyr Glu Pro
       1170                1175                1180
   acg tcc ggg cgc gtg ctc ctg gac ggc aag gac gtg cgc aag tac aac     3600
   Thr Ser Gly Arg Val Leu Leu Asp Gly Lys Asp Val Arg Lys Tyr Asn
   1185                1190                1195                1200
   ctg cgg gcg ctg cgg cgc gtg gtg gcg gtg gcg ccg cag gag ccg ttc     3648
   Leu Arg Ala Leu Arg Arg Val Val Ala Val Ala Pro Gln Glu Pro Phe
                   1205                1210                1215
   ctg ttc gcg gcg agc atc cac gac aac atc gcg tac ggg cgc gag ggc     3696
   Leu Phe Ala Ala Ser Ile His Asp Asn Ile Ala Tyr Gly Arg Glu Gly
               1220                1225                1230
   gcg acg gag gcg gag gtg gtg gag gcg gcg acg cag gcg aac gcg cac     3744
   Ala Thr Glu Ala Glu Val Val Glu Ala Ala Thr Gln Ala Asn Ala His
           1235                1240                1245
   cgg ttc atc gcg gcg ctg ccg gag ggc tac ggg acg cag gtg ggc gag     3792
   Arg Phe Ile Ala Ala Leu Pro Glu Gly Tyr Gly Thr Gln Val Gly Glu
       1250                1255                1260
   cgc ggg gtg cag ctg tcg ggc ggg cag cgg cag cgg atc gcg atc gcg     3840
   Arg Gly Val Gln Leu Ser Gly Gly Gln Arg Gln Arg Ile Ala Ile Ala
   1265                1270                1275                1280
   cgc gcg ctg gtg aag cag gcg gcc atc gtg ctg ctg gac gag gcg acc     3888
   Arg Ala Leu Val Lys Gln Ala Ala Ile Val Leu Leu Asp Glu Ala Thr
                   1285                1290                1295
   agc gcg ctg gac gcc gag tcg gag cgg tgc gtg cag gag gcg ctg gag     3936
   Ser Ala Leu Asp Ala Glu Ser Glu Arg Cys Val Gln Glu Ala Leu Glu
               1300                1305                1310
   cgc gcg ggg tcc ggg cgc acc acc atc gtg gtg gcg cac cgg ctg gcc     3984
   Arg Ala Gly Ser Gly Arg Thr Thr Ile Val Val Ala His Arg Leu Ala
           1315                1320                1325
   acg gtg cgc ggc gcg cac acc atc gcg gtc atc gac gac ggc aag gtg     4032
   Thr Val Arg Gly Ala His Thr Ile Ala Val Ile Asp Asp Gly Lys Val
       1330                1335                1340
   gcg gag cag ggg tcg cac tcg cac ctg ctc aag cac cat ccc gac ggg     4080
   Ala Glu Gln Gly Ser His Ser His Leu Leu Lys His His Pro Asp Gly
   1345                1350                1355                1360
   tgc tac gcg cgg atg ctg cag ctg cag cgg ctg acg ggc ggg tgc cgc    4128
   Cys Tyr Ala Arg Met Leu Gln Leu Gln Arg Leu Thr Gly Gly Cys Arg
                   1365                1370                1375
   gcc cgg gcc gcc gcc gtc gtc gtc caa cgg ggc cgc cgc gta gga tgg    4176
   Ala Arg Ala Ala Ala Val Val Val Gln Arg Gly Arg Arg Val Gly Trp
               1380                1385                1390
   atg gat gga tca tgg atg agt ttg gtt cct tgataaa                    4213
   Met Asp Gly Ser Trp Met Ser Leu Val Pro
           1395                1400
<210> 9
   <211> 1402
   <212> PRT
   <213> Sorghum bicolor
<400> 9
   Met Ser Thr Asn Asp Pro Asp Glu Ile Arg Ala Arg Val Val Val Leu
   1               5                   10                  15
   Gly Ala Pro His Ala Asp Asp Asp Ala Gly Asp Glu Trp Ala Arg Pro
               20                  25                  30
   Glu Leu Glu Ala Phe His Leu Pro Ser Pro Ala His Gln Pro Pro Gly
           35                  40                  45
   Phe His Leu Ala Ala Gly His Gln Pro Glu Ala Ala Ala Glu Gln Pro
       50                  55                  60
   Thr Thr Leu Pro Ala Ala Arg Arg Thr Ser Asp Thr Ser Thr Ala Ala
   65                  70                  75                  80
   Gly Ala Ala Pro Pro Ser Pro Ser Pro Pro Pro Pro Pro Ala Pro Leu
                   85                  90                  95
   Glu Met Asp Gln Pro Pro Asn Ala Lys Pro Ala Ser Ser Ser Ala Ala
               100                 105                 110
   Ala Ala Gly Ala Asn Asp Asn Lys Lys Pro Thr Pro Pro Ala Ala Leu
           115                 120                 125
   Arg Asp Leu Phe Arg Phe Ala Asp Gly Leu Asp Cys Ala Leu Met Leu
       130                 135                 140
   Val Gly Thr Leu Gly Ala Leu Val His Gly Cys Ser Leu Pro Val Phe
   145                 150                 155                 160
   Leu Arg Phe Phe Ala Asp Leu Val Asp Ser Phe Gly Ser His Ala Asn
                   165                 170                 175
   Asp Pro Asp Thr Met Val Arg Leu Val Val Lys Tyr Ala Phe Tyr Phe
               180                 185                 190
   Leu Val Val Gly Ala Ala Ile Trp Ala Ser Ser Trp Ala Glu Ile Ser
           195                 200                 205
   Cys Trp Met Trp Thr Gly Glu Arg Gln Ser Thr Arg Met Arg Ile Arg
       210                 215                 220
   Tyr Leu Asp Ala Ala Leu Arg Gln Asp Val Ser Phe Phe Asp Thr Asp
   225                 230                 235                 240
   Val Arg Thr Ser Asp Val Ile Tyr Ala Ile Asn Ala Asp Ala Val Val
                   245                 250                 255
   Gly Ala Gly Arg His Gln Arg Glu Ala Gly Gln Pro His Pro Leu His
               260                 265                 270
   Gly His Leu Arg Gly Gly Leu Arg Arg Gly Leu His Arg Arg Leu Ala
           275                 280                 285
   Ala Gly Ala Arg His Ala Arg Arg Arg Ala Ala His Arg Arg His Arg
       290                 295                 300
   Gly Ala Gln Arg Arg Arg Ala Arg Gln Ala Leu Leu Gln Glu Pro Gly
   305                 310                 315                 320
   Arg Ala Val Gly Arg Gln Arg His Arg Gly Ala Gly Ala Arg Ala Asp
                   325                 330                 335
   Thr Asp Arg Ala Gly Leu Arg Arg Arg Gly Ala Arg Asn Ala Gly Val
               340                 345                 350
   Leu Gly Gly Val Gly Arg Arg Ala Glu Asp Arg Leu Pro Gln Arg Leu
           355                 360                 365
   Arg Gln Gly Ala Arg Pro Arg Arg His Leu Leu His Arg Leu Leu Leu
       370                 375                 380
   Leu Arg Pro Pro Ala Leu Val Arg Arg Thr Pro Arg Pro Arg Asn His
   385                 390                 395                 400
   Thr Asn Gly Gly Leu Ala Ile Ala Thr Met Phe Ser Val Met Ile Gly
                   405                 410                 415
   Gly Leu Ala Leu Gly Gln Ser Ala Pro Ser Met Ala Ala Phe Ala Lys
               420                 425                 430
   Ala Arg Val Ala Ala Ala Lys Ile Phe Arg Ile Ile Asp His Arg Pro
           435                 440                 445
   Gly Ile Ser Ser Arg Asp Gly Glu Asp Gly Gly Gly Val Glu Leu Glu
       450                 455                 460
   Ser Val Thr Gly Arg Val Glu Met Arg Gly Val Asp Phe Ala Tyr Pro
   465                 470                 475                 480
   Ser Arg Pro Asp Val Pro Ile Leu Arg Gly Phe Ser Leu Ser Val Pro
                   485                 490                 495
   Ala Gly Lys Thr Ile Ala Leu Val Gly Ser Ser Gly Ser Gly Lys Ser
               500                 505                 510
   Thr Val Val Ser Leu Leu Glu Arg Phe Tyr Asp Pro Ser Ala Gly Gln
           515                 520                 525
   Ile Leu Leu Asp Gly His Asp Leu Lys Ser Leu Lys Leu Arg Trp Leu
       530                 535                 540
   Arg Gln Gln Ile Gly Leu Val Ser Gln Glu Pro Thr Leu Phe Ala Thr
   545 550 555 560
   Ser Ile Lys Glu Asn Leu Leu Leu Gly Arg Asp Ser Gln Ser Ala Thr
   565 570 575
   Gln Ala Glu Met Glu Glu Ala Ala Arg Val Ala Asn Ala His Ser Phe
   580 585 590
   Ile Val Lys Leu Pro Asp Gly Tyr Asp Thr Gln Val Gly Glu Arg Gly
   595 600 605
   Leu Gln Leu Ser Gly Gly Gln Lys Gln Arg Ile Ala Ile Ala Arg Ala
   610 615 620
   Met Leu Lys Asn Pro Ala Ile Leu Leu Leu Asp Glu Ala Thr Ser Ala
   625 630 635 640
   Leu Asp Ser Glu Ser Glu Lys Leu Val Gln Glu Ala Leu Asp Arg Phe
   645 650 655
   Met Ile Gly Arg Thr Thr Leu Val Ile Ala His Arg Met Ser Thr Ile
   660 665 670
   Arg Lys Ala Asp Val Val Ala Val Leu Gln Gly Gly Pro Val Ser Glu
   675 680 685
   Met Gly Ala His Asp Glu Leu Met Ala Lys Gly Glu Asn Gly Thr Tyr
   690 695 700
   Ala Lys Phe Ile Arg Met Gln Glu Gln Ala His Glu Ala Ala Phe Val
   705 710 715 720
   Asn Ala Arg Arg Ser Ser Ala Arg Pro Ser Ser Ala Arg Asn Ser Val
   725 730 735
   Ser Ser Pro Ile Met Thr Arg Asn Ser Ser Tyr Gly Arg Ser Pro Tyr
   740 745 750
   Ser Arg Arg Leu Ser Asp Phe Ser Thr Ser Asp Phe Thr Leu Ser Ile
   755 760 765
   His Asp Pro His His His His Arg Thr Met Ala Asp Lys Gln Leu Ala
   770 775 780
   Phe Arg Ala Gly Ala Ser Ser Phe Leu Arg Leu Ala Arg Met Asn Ser
   785 790 795 800
   Pro Glu Trp Ala Tyr Ala Leu Val Gly Ser Leu Gly Ser Met Val Cys
   805 810 815
   Gly Ser Phe Ser Ala Ile Phe Ala Tyr Ile Leu Ser Ala Val Leu Ser
   820 825 830
   Val Tyr Tyr Ala Pro Asp Pro Arg Tyr Met Lys Arg Glu Ile Ala Lys
   835 840 845
   Tyr Cys Tyr Leu Leu Ile Gly Met Ser Ser Ala Ala Leu Leu Phe Asn
   850 855 860
   Thr Val Gln His Val Phe Trp Asp Thr Val Gly Glu Asn Leu Thr Lys
   865 870 875 880
   Arg Val Arg Glu Lys Met Phe Ala Ala Val Leu Arg Asn Glu Ile Ala
   885 890 895
   Trp Phe Asp Ala Asp Glu Asn Ala Ser Ala Arg Val Ala Ala Arg Leu
   900 905 910
   Ala Leu Asp Ala Gln Asn Val Arg Ser Ala Ile Gly Asp Arg Ile Ser
   915 920 925
   Val Ile Val Gln Asn Ser Ala Leu Met Leu Val Ala Cys Thr Ala Gly
   930 935 940
   Phe Val Leu Gln Trp Arg Leu Ala Leu Val Leu Leu Ala Val Phe Pro
   945 950 955 960
   Leu Val Val Ala Ala Thr Val Leu Gln Lys Met Phe Met Lys Gly Phe
   965 970 975
   Ser Gly Asp Leu Glu Ala Ala His Ala Arg Ala Thr Gln Ile Ala Gly
   980 985 990
   Glu Ala Val Ala Asn Leu Arg Thr Val Ala Ala Phe Asn Ala Glu Arg
   995 1000 1005
   Lys Ile Thr Gly Leu Phe Glu Ala Asn Leu Arg Gly Pro Leu Arg Arg
   1010 1015 1020
   Cys Phe Trp Lys Gly Gln Ile Ala Gly Ser Gly Tyr Gly Val Ala Gln
   1025 1030 1035 1040
   Phe Leu Leu Tyr Ala Ser Tyr Ala Leu Gly Leu Trp Tyr Ala Ala Trp
   1045 1050 1055
   Leu Val Lys His Gly Val Ser Asp Phe Ser Arg Thr Ile Arg Val Phe
   1060 1065 1070
   Met Val Leu Met Val Ser Ala Asn Gly Ala Ala Glu Thr Leu Thr Leu
   1075 1080 1085
   Ala Pro Asp Phe Val Lys Gly Gly Arg Ala Met Arg Ser Val Phe Glu
   1090 1095 1100
   Thr Ile Asp Arg Lys Thr Glu Val Glu Pro Asp Asp Val Asp Ala Ala
   1105 1110 1115 1120
   Pro Val Pro Glu Arg Pro Lys Gly Glu Val Glu Leu Lys His Val Asp
   1125 1130 1135
   Phe Ser Tyr Pro Ser Arg Pro Asp Ile Gln Val Phe Arg Asp Leu Ser
   1140 1145 1150
   Leu Arg Ala Arg Ala Gly Lys Thr Leu Ala Leu Val Gly Pro Ser Gly
   1155 1160 1165
   Cys Gly Lys Ser Ser Val Leu Ala Leu Val Gln Arg Phe Tyr Glu Pro
   1170 1175 1180
   Thr Ser Gly Arg Val Leu Leu Asp Gly Lys Asp Val Arg Lys Tyr Asn
   1185 1190 1195 1200
   Leu Arg Ala Leu Arg Arg Val Val Ala Val Ala Pro Gln Glu Pro Phe
                   1205                1210                1215
   Leu Phe Ala Ala Ser Ile His Asp Asn Ile Ala Tyr Gly Arg Glu Gly
               1220                1225                1230
   Ala Thr Glu Ala Glu Val Val Glu Ala Ala Thr Gln Ala Asn Ala His
           1235                1240                1245
   Arg Phe Ile Ala Ala Leu Pro Glu Gly Tyr Gly Thr Gln Val Gly Glu
       1250                1255                1260
   Arg Gly Val Gln Leu Ser Gly Gly Gln Arg Gln Arg Ile Ala Ile Ala
   1265                1270                1275                1280
   Arg Ala Leu Val Lys Gln Ala Ala Ile Val Leu Leu Asp Glu Ala Thr
                   1285                1290                1295
   Ser Ala Leu Asp Ala Glu Ser Glu Arg Cys Val Gln Glu Ala Leu Glu
               1300                1305                1310
   Arg Ala Gly Ser Gly Arg Thr Thr Ile Val Val Ala His Arg Leu Ala
           1315                1320                1325
   Thr Val Arg Gly Ala His Thr Ile Ala Val Ile Asp Asp Gly Lys Val
       1330                1335                1340
   Ala Glu Gln Gly Ser His Ser His Leu Leu Lys His His Pro Asp Gly
   1345                1350                1355                1360
   Cys Tyr Ala Arg Met Leu Gln Leu Gln Arg Leu Thr Gly Gly Cys Arg
                   1365                1370                1375
   Ala Arg Ala Ala Ala Val Val Val Gln Arg Gly Arg Arg Val Gly Trp
               1380                1385                1390
   Met Asp Gly Ser Trp Met Ser Leu Val Pro
           1395                1400

## Description

### FIELD OF THE INVENTION

The present invention relates to the genetic manipulation of organisms, particularly plants, with genes that control growth and development. The invention further relates to genes that control growth, including homologues and mutant forms, the proteins encoded therefrom and plants transformed with these genes.

### BACKGROUND OF THE INVENTION

Dwarf plants have had a major impact on agriculture. Dwarf varieties of wheat are widely used in North America due to both reduced potential for lodging and high yields. Dwarf fruit trees are also extensively used and allow farmers to produce more fruit per acre thereby increasing economic yield potential. There are other benefits that may be realized from the use of dwarf crop plants and dwarf fruit trees including reductions in the amounts of pesticides and fertilizers required, higher planting densities and reduced labor costs.

In view of the current trends of both increasing human population and the decreasing land area suitable for agriculture, increasing agricultural productivity is, and will continue to be, a challenge of paramount importance. Dwarf crop plants and fruit trees have been and will continue to be important components of our agricultural production system. Increased usage of dwarf crop plants and dwarf fruit trees may help to meet the agricultural production demands of the future. However, commercially acceptable dwarf varieties are not available for all crops.

In addition to the use of dwarf plants to control plant height, synthetic chemicals are routinely applied to certain economically important plant species to reduce growth. Plant growth regulators known as growth retardants are used to reduce stem elongation in a variety of crops including cotton, grape vines, fruit trees, peanuts, wheat and ornamentals such as azaleas, chrysanthemums, hydrangeas, poinsettias and many bedding plants. All of the commonly used growth retardants are inhibitors of gibberellin biosynthesis and limit stem or shoot growth by reducing elongation. In the United States, the most widely used growth retardant is mepiquat chloride, which is registered for use on cotton. Benefits attributed to the use of mepiquat chloride on cotton include increased yield, improved defoliation, improved stress tolerance, more uniform crop maturity and the ability to harvest earlier. Previously, the growth retardant daminozide was registered for use in the United States on apples, grapes and peanuts under the trademarks ALAR and KYLAR but was removed from use on food crops due to human health concerns. Despite the demands of agricultural producers for a product to replace diaminozide, there are no growth retardants registered for use on grapes, fruit trees and peanuts in the United States. Daminozide, however, is still widely used on certain non-food, plant species.

Uncovering the molecular mechanisms that control plant growth processes such as cell division and cell elongation will likely aid in the development of new plant varieties with reduced stature and new methods for reducing plant growth. Such new plant varieties and methods may provide both farmers and horticulturists with environmentally benign alternatives to the use of synthetic growth-retarding chemicals.

Elongation of plant cells and organs is one of the most critical parameters of plant growth and development. Regulation of this trait in plants, however, is a fairly complicated process, as both external and internal factors influence it. The most important external stimulus is light, with its normally repressible or negative effect on cell elongation (Quail, P.H. (1995) Science 268:675-680; Kende et al. (1997) Plant Cell 9:1197-1210). The internal control of cell elongation is mediated by a number of chemicals, normally referred to as plant growth regulators or hormones (Kende et al. (1997) Plant Cell 9:1197-1210). Among the classical plant hormones, auxins and gibberellins (GAs) both promote cell elongation whereas cytokinins and abscisic acid each have been shown to have a negative effect on cell elongation (Kende et al. (1997) Plant Cell 9:1197-1210). Recently, another class of plant growth regulators, named brassinosteroids, has been identified that also dramatically promote plant growth (Yokota, T. (1997) Trends Plant Sci. 2:137-143; Azpiroz et al. (1998) Plant Cell 10:219-230; Choe et al. (1998) Plant Cell 10:231-243). However, the mechanisms by which plant hormones act, either singly or in concert, to control cell elongation remains unclear.

One way to gain an understanding of mechanisms that mediate cell elongation is to study mutants in which this aspect of plant growth is compromised (Klee et al. (1991) Annu. Rev. Plant Physiol. Plant Mol. Biol. 42:529-551). Numerous such mutants have been identified across most plant species, including maize, in which more than 25 single-gene mutations that affect plant stature have been characterized (Coe et al. (1988) In: Corn & Corn Improvement. G. F. Sprague (Ed.) Madison, WI ; Sheridan, W.F. (1988) Annu. Rev. Genet. 22:353-385). These dwarf mutants are considered to be GA related, mainly because GA is the only phytohormone whose role in regulating height in maize has been convincingly established (Phinney et al. (1985) Curr. Top. Plant Biochem. Physiol. 4:67-74; Fujioka et al. (1988) Proc. Natl. Acad. Sci. USA 85:9031-9035). Both types of mutants, GA responsive and GA non-responsive, have been found in this collection of maize mutants. While genes for a number of GA-responsive mutants have been cloned and found to be involved in GA biosynthesis (Bensen et al. (1995) Plant Cell 7:75-84; Winkler et al. (1995) Plant Cell 7:1307-1317), nothing is known about the nature of defects in GA non-responsive maize mutants.

One type of GA non-responsive dwarf mutants that have received much attention from maize geneticists and breeders is called brachytic. These dwarfs are characterized by internodes of substantially reduced length, relative to wild-type, without having any effect on the size or number of other organs, including the leaves, ear and tassel (Kempton, J.H. (1920) J. Hered 11:111-115). There are three known brachytic mutations in maize, *br1, br2* and *br3,* all of which are recessive (Coe et al. (1988) In: Corn & Corn Improvement, G. F. Sprague (Ed.) Madison, WI; Sheridan, W.F. (1988) Annu. Rev. Genet. 22:353-385). Because of the commercial interest in *br2* for enhancing plant productivity (Pendleton et al. (1961) Crop Sci. 1:433-435; Duvick, D.N. (1977) Maydica 22:187-196; Djisbar et al. (1987) Maydica 32:107-123; Russel, W.A. (1991) Adv. Agron. 46:245-298), this dwarf has been characterized the most. Depending on the genetic background, plants homozygous recessive for *br2* are 30-70% shorter than their normal sibs. This reduction in plant height is exclusively due to a reduction of the length of stalk (stem) internodes. In addition to being dwarf, *br2* mutants grown under greenhouse conditions often suffer from buggy whip, a disease-like condition in which the unfurling leaves in the whorl undergo necrosis and stay stuck together. This condition often results in the death of the growing tip of the plant.

Although the dwarfing trait in maize has been extensively studied both genetically and molecularly, it has yet to be exploited successfully in breeding efforts in this crop plant. In contrast, dwarf mutants of sorghum have contributed significantly to the development of modern day cultivars. Sorghum and maize are both members of the grass (Poaceae or Gramineae) family and thus share many characteristics including genomic organization and plant body form. Out of the four dwarfing mutations exploited in sorghum, *dw3,* whose dwarfing phenotype looks very similar to that of *br2* in maize, appears to be the most prominent. However, the only *dw3* allele (*dw3-ref*) available thus far has a serious problem which limits its agronomic value. The dwarf phenotype associated with the *dw3* allele is unstable, with a reversion frequency to wild-type (tall) as high as about 1% in certain genetic backgrounds. The instability of this dwarf phenotype, the mechanism of which has eluded sorghum geneticists thus far, not only continues to embarrass sorghum breeders, but also sometimes leads to the rejection of an otherwise promising inbred or hybrid.

To keep up with the demand for increased agricultural production, new targets are needed for genetically engineering agricultural plants for the improvement of agronomic characteristics. Elucidating the molecular mechanisms of cell division and elongation will provide new targets for agricultural scientists to manipulate.

### SUMMARY OF THE INVENTION

Compositions and methods for expressing genes encoding P-glycoproteins of the invention in plants are provided. In particular, the invention provides nucleotide sequences of the *Dw3* gene of sorghum. The sequences of the invention are useful in transforming plants for tissue-preferred or constitutive expression and find use in methods for controlling the growth of organisms, particularly stem growth in plants.
Therefore the invention provides:
- an isolated nucleic acid molecule comprising a nucleotide sequence selected from the group consisting of:
   (a) a nucleotide sequence set forth in SEQ ID NO: 1;
   (b) a nucleotide sequence set forth in SEQ ID NO: 2;
   (c) a nucleotide sequence set forth in SEQ ID NO: 3;
   (d) a nucleotide sequence set forth in SEQ ID NO: 7;
   (e) a nucleotide sequence set forth in SEQ ID NO: 8;
   (f) a nucleotide sequence consisting of at least 150 contiguous nucleotides of the nucleotide sequence set forth in any one of (a)-(e), wherein said nucleotide sequence encodes a P-glycoprotein that controls plant growth;
   (g) a nucleotide sequence encoding the amino acid sequence set forth in SEQ ID NO: 9;
   (h) a nucleotide sequence encoding at least 70 contiguous amino acids of the amino acid sequence set forth in SEQ ID NO: 9, wherein said nucleotide sequence encodes a P-glycoprotein that controls plant growth;
   (i) a nucleotide sequence comprising at least 80% identity to the sequence set forth in SEQ ID NO: 7, wherein said nucleotide sequence encodes a P-glycoprotein that controls plant growth;
   (j) a nucleotide sequence comprising at least 80% identity to the sequence set forth in SEQ ID NO: 8, wherein said nucleotide sequence encodes a P-glycoprotein that controls plant growth;
   (k) a nucleotide sequence that is complementary to the nucleotide sequence of any one of (a)-(k); and
   (l) a nucleotide sequence that hybridizes under stringent conditions to the nucleotide sequence any one of (a)-(e), or to a complementary sequence thereof, wherein said stringent conditions comprise hybridization in a solution comprising 50% formamide, 1 M NaCl, 1% SDS at 37°C and a wash in 0.1X SSC at 60°C, and said nucleotide sequence encodes a P-glycoprotein that controls plant growth;
- a method for modifying the growth of a plant, said method comprising transforming a plant with a nucleotide sequence encoding a P-glycoprotein wherein said P-glycoprotein functions to control growth of an organism, said nucleotide sequence operably linked for the production of antisense transcripts to a promoter capable of driving the expression of said sequence in said plant; wherein said nucleotide sequence is as defined by the invention; and
- a method for identifying a sorghum plant having a stable dwarf phenotype comprising:
   (a) obtaining a dwarf sorghum plant which is a descendent of an unstable *dw3* dwarf sorghum plant.
   (b) analyzing the genomic DNA from said dwarf sorghum plant to determine if at least one copy of a *dw3* gene lacks an insertion, or at least a portion of said insertion;
   (c) selecting said dwarf sorghum plant if said dwarf sorghum plant comprises in its genome at least one copy of said *dw3* gene lacking said insertion, or lacking said portion thereof.

Methods are provided for identifying plants that possess a mutant allele that is capable of conferring a stable mutant phenotype on an organism. Such methods find use in agriculture, particularly in the breeding of dwarf crop plants, particularly dwarf sorghum plants.

Expression cassettes comprising the sequences of the invention are provided. Additionally provided are transformed plants, plant tissues, plant cells and seeds thereof. Isolated proteins encoded by the nucleotide sequences of the invention are provided. Therefore the invention also provides:
- an isolated protein comprising a member selected from the group consisting of:
   (a) a polypeptide comprising the amino acid sequence set forth in SEQ ID NO: 9;
   (b) a polypeptide comprising at least 70 contiguous amino acids of the amino acid sequence set forth in SEQ ID NO: 9, wherein said polypeptide is a P-glycoprotein that controls plant growth;
   (c) a polypeptide encoded by the nucleotide sequence set forth in SEQ ID NO: 8; and
   (d) a polypeptide encoded by an amino acid sequence comprising at least 80% identity to the amino sequence of (a), wherein said polypeptide is a P-glycoprotein that controls plant growth.

### DETAILED DESCRIPTION OF THE INVENTION

The present invention relates, in particular, to controlling stem growth in plants. Thus, transformed plants, plant cells, plant tissues and seeds comprising nucleic acids of the invention are provided.

Compositions of the invention include the native nucleotide sequences for the P-glycoprotein gene of the sorghum *Dw3* locus and the respective amino acid sequence for the P-glycoproteins encoded thereby, as claimed. The *Dw3* nucleotide sequences are set forth in SEQ ID NOS: 1-3 and 7-8. The nucleotide sequences or corresponding antisense sequences find use in modulating the expression of a P-glycoprotein in a plant or plant cell. That is, the coding sequences can be used to increase the expression while antisense sequences can be used to decrease expression.

The sequences of the invention find use in methods of modifying plant growth. Toward this end, the sequences of the invention may be utilized in expression cassettes or nucleotide constructs operably linked to any one of a variety of plant promoters. Aspects of plant growth that may be impacted by the methods of the invention include, but are not limited to, plant height; the size, shape and number of cells and organs; cell division rate; cell elongation rate; the growth rate of the plant, its organs, tissues and cells; timing and location of organ initiation; life span; and the like.

The invention discloses methods for reducing plant growth which find use as alternatives to applying synthetic, growth-retarding chemicals to plants. These methods provide environmentally safe alternatives to traditional means of retarding stem elongation or growth with synthetic chemicals. Some embodiments of the invention make use of plants transformed with tissue-preferred promoters, particularly stem-preferred promoters, operably linked to nucleotide sequences of the invention.

Methods are provided for reducing the growth of a plant. Such methods involve transforming plants with at least one nucleotide sequence of the invention. The nucleotide sequences may be used in either the sense or antisense orientation to suppress the level of an endogenous P-glycoprotein that controls the growth of a plant. By reducing the level in a plant of such a P-glycoprotein, particularly one that controls stem or stalk growth, a plant of reduced stature, a dwarf plant, may be achieved. Dwarf plants having improved agronomic characteristics can be obtained by these methods. Such improved agronomic characteristics include, but are not limited to, reduced potential for lodging, increased water-use efficiency, reduced life cycle, increased harvest efficiency and increased yield per unit area. The methods of the invention can eliminate the need to graft shoots of fruit trees on dwarfing rootstocks to produce dwarf fruit trees.

The methods of the invention find use in producing dwarf varieties of crop plants. In one embodiment of the invention, a dwarf Basmati rice plant is produced by transforming the plant with a nucleotide sequence of the invention. Basmati rice, known for its aromatic fragrance, slender, elongated grains, and relatively short cooking time, is the favorite type of rice of the majority of people in the Indian sub-continent. While commercially acceptable dwarf cultivars have been developed for other types of rice, previous attempts to produce commercially acceptable varieties of Basmati rice by traditional plant breeding methods have failed. While dwarf plants were obtained in such attempts, some of the distinctive grain characteristics that consumers expect in Basmati rice were not retained in the dwarf plants. The methods of the invention provide a means of making dwarf Basmati rice plants that produce grain possessing the characteristics desired by consumers.

The desired dwarf Basmati rice plants are produced by transforming a non-dwarf Basmati rice plant with a nucleotide sequence of the invention operably linked to a promoter that drives expression in a plant. While the choice of promoter depends on the desired outcome, the preferred promoters are tissue-preferred promoters, particularly stem-preferred promoters. Through cosuppression (sense suppression) or antisense suppression, such plants produce reduced levels of at least one P-glycoprotein that controls the growth of the Basmati rice plant, particularly stem growth.

Also provided are methods for identifying a sorgham plant with a stable dwarf phenotype. Such methods find use in agriculture, particularly in the development of improved crop plants. The methods relate to an insertion-induced, mutant phenotype. By "insertion-induced, mutant phenotype" is intended a mutant phenotype that is due to the insertion of a nucleotide, or a sequence of nucleotides, into the sequence of a gene of interest. While the invention does not depend upon a particular genetic mechanism for such an insertion-induced mutant phenotype, the presence of such an insertion within a gene typically disrupts the normal wild-type function of the gene, or gene product thereof. While the methods of the invention are not bound by any particular type of insertion, such an insertion may be due to, for example, the insertion of a transposon or transposable element, or the duplication of a nucleotide sequence such as those which are known to occur as a result of genetic recombination.

Preferably, such an insertion-induced phenotype is unstable from one generation to the next. That is, self pollination of one or more like plants having the insertion-induced phenotype results in at least one individual from among the resulting progeny population that has reverted to the wild-type phenotype. More preferably, such phenotypic instability, from one generation to the next, is due to the loss of at least a portion of the insertion from the gene of interest and that such a loss results in at least one progeny plant, which has reverted to a wild-type phenotype. The methods of the present invention involve identifying an individual with a stable mutant phenotype from among such progeny population.

To identify a plant possessing an allele of a gene that confers a stable mutant phenotype, genomic DNA from a mutant plant is analyzed to determine if at least one copy of the gene of interest lacks the insertion, or at least a portion thereof. Generally, the mutant plant is selected from a population of progeny derived from the self pollination of one or more plants having the insertion-induced, mutant phenotype. Typically, in a population of such progeny, wild-type revertants will also be observed, indicating that at least a portion of the insertion has excised from the gene of interest. The genomic DNA of the selected mutant plant can be isolated and analyzed for the absence of all or a portion of the insertion by techniques known to those of ordinary skill in the art such as, for example, Southern blotting, restriction fragment length polymorphism (RFLP) analysis and DNA amplification by polymerase chain reaction (PCR). Once a mutant plant lacking a portion of the insertion is identified, the progeny of such a mutant plant can be monitored to verify phenotypic stability. If desired, subsequent generations can also be monitored.

A method of the invention involves identifying a sorghum plant with a stable dwarf phenotype. Such a sorghum plant possesses in its genome a stable mutant allele of the *Dw3* gene. Such a stable mutant allele is capable of conferring a stable dwarf phenotype on a sorghum plant and the nucleotide sequence of a fragment of such an allele is set forth in SEQ ID NO: 2. One method of the invention employs RFLP analysis utilizing Southern blotting with a probe derived from nucleotide sequences of maize *Br2.* This method additionally involves PCR amplification and DNA sequence analysis to determine the nucleotide sequence of the stable mutant allele.

The P-glycoproteins of the invention encompass all polypeptides and nucleotide sequences of the invention whether or not such polypeptides possess covalently attached carbohydrates or carbohydzate-containing chains.

By "mutant phenotype" is intended any non-wild-typs, non-typical or non-standard phenotype which occurs as a result of a genetic alteration in the genome of an organism. When used in reference to domesticated plants and animals, a "mutant phenotype" is any phenotype that is substantially different from the typical phenotype of the particular domesticated breed or cultivated variety from which the mutant phenotype arose.

By "mutant plant" is intended a plant having a mutant phenotype.

By "mutant allele" is intended an allele of a gene that is capable of causing a "mutant phenotype."

By "dwarf" is intended atypically small. By "dwarf plant" is intended an atypically small plant. Generally, such a "dwarf plant" has a stature or height that is reduced from that of a typical plant by about 5%, 10%, 15%, 20%, 25%, 30%, 35%, 40%, 45%, 50%, 55%, 60% or greater. Generally, but not exclusively, such a dwarf plant is characterized by a reduced stem, stalk or trunk length when compared to the typical plant.

By "nucleotide molecule" is intended a molecule composed of nucleotides covalently bound to one another. Nucleotides include both ribonucleotides and deoxyribonucleotides. "Nucleotide molecule" encompasses single-stranded and double stranded forms of both DNA and RNA. "Nucleotide molecules" may be naturally occurring, synthetic or a combination of both. The linear arrangement of nucleotides in a "nucleotide molecule" is referred to as a "nucleotide sequence" and unless specified otherwise is presented herein from left to right corresponding to 5'-to-3' direction. Because of the complementary nature of the opposite strands of a double-stranded nucleotide molecule, a nucleotide sequence of the invention additionally encompasses its complementary antisense sequence.

Compositions of the invention include native nucleotide sequences for genes encoding multidrug-resistance-like-gene-encoded P-glycoproteins, homologues of multidrug-resistance-like-gene-encoded P-glycoproteins, antisense sequences, as well as fragments and variants and fragments thereof. In particular, the present invention provides for isolated nucleic acid molecules comprising nucleotide sequences encoding the amino acid sequences shown in SEQ ID NOS: 4 and 9.

Further provided are polypeptides having an amino acid sequence encoded by a nucleic acid molecule described herein, for example those set forth in SEQ ID NOS: 3 and 8, respectively as claimed.

The invention encompasses isolated or substantially purified nucleic acid or protein compositions. An "isolated" or "purified" nucleic acid molecule or protein, or biologically active portion thereof, is substantially free of other cellular material, or culture medium when produced by recombinant techniques, or substantially free of chemical precursors or other chemicals when chemically synthesized. Preferably, an "isolated" nucleic acid is free of sequences (preferably protein encoding sequences) that naturally flank the nucleic acid (i.e., sequences located at the 5' and 3' ends of the nucleic acid) in the genomic DNA of the organism from which the nucleic acid is derived. For example, in various embodiments, the isolated nucleic acid molecule can contain less than about 5 kb, 4 kb, 3 kb, 2 kb, 1 kb, 0.5 kb, or 0.1 kb of nucleotide sequences that naturally flank the nucleic acid molecule in genomic DNA of the cell from which the nucleic acid is derived. A protein that is substantially free of cellular material includes preparations of protein having less than about 30%, 20%, 10%, 5%, (by dry weight) of contaminating protein. When the protein of the invention or biologically active portion thereof is recombinantly produced, preferably culture medium represents less than about 30%, 20%, 10%, or 5% (by dry weight) of chemical precursors or non-protein-of-interest chemicals.

Fragments and variants of the disclosed nucleotide sequences and proteins encoded thereby are also encompassed by the present invention. By "fragment" is intended a portion of the nucleotide sequence or a portion of the amino acid sequence and hence protein encoded thereby. Fragments of a nucleotide sequence may encode protein fragments that retain biological activity of the native P-glycoprotein of the invention and hence retain one or more functions of the native P-glycoprotein such as, for example, transmembrane transporter activity and ATP binding. Alternatively, fragments of a nucleotide sequence that are useful as hybridization probes may or may not encode protein fragments retaining biological activity. Thus, fragments of a nucleotide sequence may range from at least about 20 nucleotides, about 50 nucleotides, about 100 nucleotides, and up to the full-length nucleotide sequence of the invention.

A fragment of a P-glycoprotein gene nucleotide sequence that encodes a biologically active portion of a P-glycoprotein of the invention wiU encode at least 70, 75, 100, 150, 200, 250, 300, 350, 400, 450, 500, 550, 600, 650, 700, 800, 900, 1,000, 1,1,00, 1,200, 1,300, or 1,400 contiguous amino acids, or up to the total number of amino acids present in a full-length P-glycoprotein of the invention (for example 1,421 amino acids for SEQ ID NO:9).

A biologically active portion of a P-glycoprotein of the invention can be prepared by isolating a portion of one of the P-glycoprotein gene nucleotide sequences of the invention, expressing the encoded portion of the P-glycoprotein e.g., by recombinant expression *in vitro*), and assessing the activity of the portion of the P-glycoprotein. Nucleic acid molecules that are fragments of a P-glycoprotein gene nucleotide sequence comprise at least 200, 300, 500, 700, 1,000, 1,200, 1,500, 2,000, 2,500, 3,000, 3,500, 4,000, 4,500, 5,000, 5,500, 6,000 nucleotides, or up to the number of nucleotides present in a full-length P-glycoprotein nucleotide sequence disclosed herein (for example, 2,139, 1,267, 1,261, 6,827, and 4213 nucleotides for SEQ ID NOS: 1-3, and 7-8, respectively).

By "variants" is intended substantially similar sequences. For nucleotide sequences, conservative variants include those sequences that, because of the degeneracy of the genetic code, encode the amino acid sequence of one of the P-glycoprotein polypeptides of the invention. Naturally occurring allelic variants such as these can be identified with the use of well-known molecular biology techniques, as, for example, with polymerase chain reaction (PCR) and hybridization techniques as outlined below. Variant nucleotide sequences also include synthetically derived nucleotide sequences, such as those generated, for example, by using site-directed mutagenesis but which still encode a P-glycoprotein protein of the invention. Generally, variants of a particular nucleotide sequence of the invention will have at least about 80%, 85%, preferably at least about 90%, 91%, 92%, 93%, 94%, 95%, 96%, 97%, and more preferably at least about 98%, 99% or more sequence identity to that particular nucleotide sequence as determined by sequence alignment programs described elsewhere herein using default parameters.

By "variant" protein is intended a protein derived from the native protein by deletion (so-called truncation) or addition of one or more amino acids to the N-terminal and/or C-terminal end of the native protein; deletion or addition of one or more amino acids at one or more sites in the native protein; or substitution of one or more amino acids at one or more sites in the native protein. Variant proteins encompassed by the present invention are biologically active, that is they continue to possess the desired biological activity of the native protein, that is, transporter activity or ATP binding activity as described herein. Such variants may result from, for example, genetic polymorphism or from human manipulation. Biologically active variants of a native P-glycoprotein of the invention will have at least about 80%, 85%, preferably at least about 90%, 91%, 92%, 93%, 94%, 95%, 96%, 97%, and more preferably at least about 98%, 99% or more sequence identity to the amino acid sequence for the native protein as determined by sequence alignment programs described elsewhere herein using default parameters. A biologically active variant of a protein of the invention may differ from that protein by as few as 1-15 amino acid residues, as few as 1-10, such as 6-10, as few as 5, as few as 4, 3, 2, or even 1, amino acid residue.

The proteins of the invention may be altered in various ways including amino acid substitutions, deletions, truncations, and insertions. Methods for such manipulations are generally known in the art. For example, amino acid sequence variants of the P-glycoproteins can be prepared by mutations in the DNA. Methods for mutagenesis and nucleotide sequence alterations are well known in the art. See for example, Kunkel (1985) Proc. Natl. Arad. Sci. USA 82:488-492; Kunkel et al. (1987) Methods in Enzymol. 154:367-382; US Patent No. 4,873,192; Walker and Gaastra, eds. (1983) Techniques in Molecular Biology (MacMillan Publishing Company, New York) and the references cited therein. Guidance as to appropriate amino acid substitutions that do not affect biological activity of the protein of interest may be found in the model of Dayhoff et al. (1978) Atlas of Protein Sequence and Structure (Natl. Biomed. Res. Found., Washington, D.C.).

Conservative substitutions, such as exchanging one amino acid with another having similar properties, may be preferred.

Thus, the genes and nucleotide sequences of the invention include both the naturally occurring sequences as well as mutant forms. Likewise, the proteins of the invention encompass both naturally occurring proteins as well as variations and modified forms thereof. Such variants will continue to possess the desired transporter activity. Obviously, the mutations that will be made in the DNA encoding the variant must not place the sequence out of reading frame and preferably will not create complementary regions that could produce secondary mRNA structure. See, EP Patent Application Publication No. 75,444.

The deletions, insertions, and substitutions of the protein sequences encompassed herein are not expected to produce radical changes in the characteristics of the protein. However, when it is difficult to predict the exact effect of the substitution, deletion, or insertion in advance of doing so, one skilled in the art will appreciate that the effect will be evaluated by routine screening assays.

Variant nucleotide sequences and proteins also encompass nucleotide sequences and proteins derived from a mutagenic and recombinogenic procedure such as DNA shuffling. With such a procedure, one or more different P-glycoprotein coding sequences can be manipulated to create a variant nucleotide sequence encoding a variant P-glycoprotein possessing the desired properties. In this manner, libraries of recombinant polynucleotides are generated from a population of related sequence polynucleotides comprising sequence regions that have substantial sequence identity and can be homologously recombined *in vitro* or *in vivo.* For example, using this approach, sequence motifs encoding a domain of interest may be shuffled between the P-glycoprotein gene of the invention and other known P-glycoprotein genes to obtain a new gene coding for a protein with an improved property of interest, such as an increased Kₘ in the case of an enzyme. Strategies for such DNA shuffling are known in the art. See, for example, Stemmer (1994) Proc. Natl. Acad. Sci. USA 91:10747-10751; Stemmer (1994) Nature 370:389-391; Crameri et al. (1997) Nature Biotech. 15:436-438; Moore et al. (1997) J. Mol. Biol. 272:336-347; Zhang et al. (1997) Proc. Natl. Acad Sci. USA 94:4504-4509; Crameri et al. (1998) Nature 391:288-291; and U.S. Patent Nos. 5,605,793 and 5,837,458.

The nucleotide sequences of the invention can be used to isolate corresponding sequences from other organisms, particularly other plants, more particularly other monocots. In this manner, methods such as PCR, hybridization, and the like can be used to identify such sequences based on their sequence homology to the sequences set forth herein. Sequences isolated based on their sequence identity to the entire sequences set forth herein or to fragments thereof are encompassed by the present invention. Such sequences include sequences that are orthologs of the disclosed sequences. By "orthologs" is intended genes derived from a common ancestral gene and which are found in different species as a result of speciation. Genes found in different species are considered orthologs when their nucleotide sequences and/or their encoded protein sequences share substantial identity as defined elsewhere herein. Functions of orthologs are often highly conserved among species.

In a PCR approach, oligonucleotide primers can be designed for use in PCR reactions to amplify corresponding DNA sequences from cDNA or genomic DNA extracted from any organism of interest. Methods for designing PCR primers and PCR cloning are generally known in the art and are disclosed in Sambrook et al. (1989) Molecular Cloning: A Laboratory Manual (2nd ed., Cold Spring Harbor Laboratory Press, Plainview, New York). See also Innis et al., eds. (1990) PCR Protocols: A Guide to Methods and Applications (Academic Press, New York); Innis and Gelfand, eds. (1995) PCR Strategies (Academic Press, New York); and Innis and Gelfand, eds. (1999) PCR Methods Manual (Academic Press, New York). Known methods of PCR include, but are not limited to, methods using paired primers, nested primers, single specific primers, degenerate primers, gene-specific primers, vector-specific primers, partially-mismatched primers, and the like.

In hybridization techniques, all or part of a known nucleotide sequence is used as a probe that selectively hybridizes to other corresponding nucleotide sequences present in a population of cloned genomic DNA fragments or cDNA fragments (*i.e.,* genomic or cDNA libraries) from a chosen organism. The hybridization probes may be genomic DNA fragments, cDNA fragments, RNA fragments, or other oligonucleotides, and may be labeled with a detectable group such as ³²P, or any other detectable marker. Thus, for example, probes for hybridization can be made by labeling synthetic oligonucleotides based on the P-glycoprotein gene nucleotide sequences of the invention. Methods for preparation of probes for hybridization and for construction of cDNA and genomic libraries are generally known in the art and are disclosed in Sambrook et al. (1989) Molecular Cloning: A Laboratory Manual (2nd ed., Cold Spring Harbor Laboratory Press, Plainview, New York).

For example, the entire *Dw3* sequence disclosed herein, or one or more portions thereof, may be used as a probe capable of specifically hybridizing to corresponding P-glycoprotein gene sequences and messenger RNAs. To achieve specific hybridization under a variety of conditions, such probes include sequences that are unique among P-glycoprotein gene sequences and are preferably at least about 10 nucleotides in length, and most preferably at least about 20 nucleotides in length. Such probes may be used to amplify corresponding P-glycoprotein gene sequences from a chosen plant by PCR. This technique may be used to isolate additional coding sequences from a desired plant or as a diagnostic assay to determine the presence of coding sequences in a plant. Hybridization techniques include hybridization screening of plated DNA libraries (either plaques or colonies; see, for example, Sambrook et al. (1989) Molecular Cloning: A Laboratory Manual (2nd ed., Cold Spring Harbor Laboratory Press, Plainview, New York).

Hybridization of such sequences may be carried out under stringent conditions. By "stringent conditions" or "stringent hybridization conditions" is intended conditions under which a probe will hybridize to its target sequence to a detectably greater degree than to other sequences (e.g., at least two-fold over background). Stringent conditions are sequence-dependent and will be different in different circumstances. By controlling the stringency of the hybridization and/or washing conditions, target sequences that are 100% complementary to the probe can be identified (homologous probing). Alternatively, stringency conditions can be adjusted to allow some mismatching in sequences so that lower degrees of similarity are detected (heterologous probing). Generally, a probe is less than about 1000 nucleotides in length, preferably less than 500 nucleotides in length.

Typically, stringent conditions will be those in which the salt concentration is less than about 1.5 M Na ion, typically about 0.01 to 1.0 M Na ion concentration (or other salts) at pH 7.0 to 8.3 and the temperature is at least about 30°C for short probes (e.g., 10 to 50 nucleotides) and at least about 60°C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulphate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C. and a wash in 0.1X SSC at 60 to 65°C. The duration of hybridization is generally less than about 24 hours, usually about 4 to about 12 hours.

Specificity is typically the function of post-hybridization washes, the critical factors being the ionic strength and temperature of the final wash solution. For DNA-DNA hybrids, the Tₘ can be approximated from the equation of Meinkoth and Wahl (1984) Anal. Biochem. 138:267-284: Tₘ = 81.5°C + 16.6 (log M) + 0.41 (%GC) - 0.61 (% form) - 500/L; where M is the molarity of monovalent cations, %GC is the percentage of guanosine and cytosine nucleotides in the DNA, % form is the percentage of formamide in the hybridization solution, and L is the length of the hybrid in base pairs. The Tₘ is the temperature (under defined ionic strength and pH) at which 50% of a complementary target sequence hybridizes to a perfectly matched probe. Tₘ is reduced by about 1 °C for each 1% of mismatching; thus, Tₘ, hybridization, and/or wash conditions can be adjusted to hybridize to sequences of the desired identity. For example, if sequences with >90% identity are sought, the Tₘ can be decreased 10°C. Generally, stringent conditions are selected to be about 5°C lower than the thermal melting point (Tₘ) for the specific sequence and its complement at a defined ionic strength and pH. However, severely stringent conditions can utilize a hybridization and/or wash at 1, 2, 3, or 4°C lower than the thermal melting point (Tₘ); moderately stringent conditions can utilize a hybridization and/or wash at 6, 7, 8, 9, or 10°C lower than the thermal melting point (Tₘ); low stringency conditions can utilize a hybridization and/or wash at 11, 12, 13, 14, 15, or 20°C lower than the thermal melting point (Tₘ). Using the equation, hybridization and wash compositions, and desired Tₘ, those of ordinary skill will understand that variations in the stringency of hybridization and/or wash solutions are inherently described. If the desired degree of mismatching results in a Tₘ of less than 45°C (aqueous solution) or 32°C (formamide solution), it is preferred to increase the SSC concentration so that a higher temperature can be used. An extensive guide to the hybridization of nucleic acids is found in Tijssen (1993) Laboratory Techniques in Biochemistry and Molecular Biology-Hybridization with Nucleic Acid Probes, Part I, Chapter 2 (Elsevier, New York); and Ausubel et al., eds. (1995) Current Protocols in Molecular Biology, Chapter 2 (Greene Publishing and Wiley-Interscience, New York). See Sambrook et al. (1989) Molecular Cloning: A Laboratory Manual (2nd ed., Cold Spring Harbor Laboratory Press, Plainview, New York).

Thus, isolated sequences that encode for P-glycoproteins and which hybridize under stringent conditions to the to the P-glycoprotein gene sequences disclosed herein, or to fragments thereof, are encompassed by the present invention. Such sequences will be at least about 80% to 85%, and even 90%, 91 %, 92%, 93%, 94%, 95%, 96%, 97%, 98%, 99% or more homologous with the disclosed sequences. That is, the sequence identity of sequences may range, sharing at least about 80% to 85%, and even at least about 75%, 80%, 85%, 90%, 91%, 92%, 93%, 94%, 95%, 96%, 97%, 98%, 99% or more sequence identity.

The following terms are used to describe the sequence relationships between two or more nucleic acids or polynucleotides: (a) "reference sequence", (b) "comparison window", (c) "sequence identity", (d) "percentage of sequence identity", and (e) "substantial identity."
(a) As used herein, "reference sequence" is a defined sequence used as a basis for sequence comparison. A reference sequence may be a subset or the entirety of a specified sequence; for example, as a segment of a full-length cDNA or gene sequence, or the complete cDNA or gene sequence.
(b) As used herein, "comparison window" makes reference to a contiguous and specified segment of a polynucleotide sequence, wherein the polynucleotide sequence in the comparison window may comprise additions or deletions (i.e., gaps) compared to the reference sequence (which does not comprise additions or deletions) for optimal alignment of the two sequences. Generally, the comparison window is at least 20 contiguous nucleotides in length, and optionally can be 30, 40, 50, 100, or longer. Those of skill in the art understand that to avoid a high similarity to a reference sequence due to inclusion of gaps in the polynucleotide sequence a gap penalty is typically introduced and is subtracted from the number of matches. Methods of alignment of sequences for comparison are well known in the art. Thus, the determination of percent identity between, any two sequences can be accomplished using a mathematical algorithm. Non-limiting examples of such mathematical algorithms are the algorithm of Myers and Miller (1988) CABIOS 4:11-17; the local homology algorithm of Smith et al. (1981) Adv. Appl. Math. 2:482; the homology alignment algorithm of Needleman and Wunsch (1970) J. Mol. Biol. 48:443-453; the search-for-similarity-method of Pearson and Lipman (1988) Proc. Natl. Acad. Sci. 85:2444-2448; the algorithm of Karlin and Altschul (1990) Proc. Natl. Acad. Sci. USA 872264, modified as in Karlin and Altschul (1993) Proc. Natl. Acad. Sci. USA 90:5873-5877.
   Computer implementations of these mathematical algorithms can be utilized for comparison of sequences to determine sequence identity. Such implementations include, but are not limited to: CLUSTAL in the PC/Gene program (available from Intelligenetics, Mountain View, California); the ALIGN program (Version 2.0) and GAP, BESTFIT, BLAST, FASTA, and TFASTA in the Wisconsin Genetics Software Package, Version 8 (available from Genetics Computer Group (GCG), 575 Science Drive, Madison, Wisconsin, USA). Alignments using these programs can be performed using the default parameters. The CLUSTAL program is well described by Higgins et al. (1988) Gene 73:237-244 (1988); Higgins et al. (1989) CABIOS 5:151-153; Corpet et al. (1988) Nucleic Acids Res. 16:10881-90; Huang et al. (1992) CABIOS 8:155-65; and Pearson et al. (1994) Meth. Mol. Biol. 24:307-331. The ALIGN program is based on the algorithm of Myers and Miller (1988) supra. A PAM120 weight residue table, a gap length penalty of 12, and a gap penalty of 4 can be used with the ALIGN program when comparing amino acid sequences. The BLAST programs of Altschul et al (1990) J. Mol. Biol. 215:403 are based on the algorithm of Karlin and Altschul (1990) supra. BLAST nucleotide searches can be performed with the BLASTN program, score = 100, wordlength = 12, to obtain nucleotide sequences homologous to a nucleotide sequence encoding a protein of the invention. BLAST protein searches can be performed with the BLASTX program, score = 50, wordlength = 3, to obtain amino acid sequences homologous to a protein or polypeptide of the invention. To obtain gapped alignments for comparison purposes, Gapped BLAST (in BLAST 2.0) can be utilized as described in Altschul et al. (1997) Nucleic Acids Res. 25:3389. Alternatively, PSI-BLAST (in BLAST 2.0) can be used to perform an iterated search that detects distant relationships between molecules. See Altschul et al. (1997) supra. When utilizing BLAST, Gapped BLAST, PSI-BLAST, the default parameters of the respective programs (e.g., BLASTN for nucleotide sequences, BLASTX for proteins) can be used. See http://www.ncbi.hlm.nih.gov. Alignment may also be performed manually by inspection.
   Unless otherwise stated, sequence identity/similarity values provided herein refer to the value obtained using GAP Version 10 using the following parameters: % identity using GAP Weight of 50 and Length Weight of 3; % similarity using Gap Weight of 12 and Length Weight of 4, or any equivalent program. By "equivalent program" is intended any sequence comparison program that, for any two sequences in question, generates an alignment having identical nucleotide or amino acid residue matches and an identical percent sequence identity when compared to the corresponding alignment generated by the preferred program.
   GAP uses the algorithm of Needleman and Wunsch (1970) J. Mol. Biol. 48: 443-453, to find the alignment of two complete sequences that maximizes the number of matches and minimizes the number of gaps. GAP considers all possible alignments and gap positions and creates the alignment with the largest number of matched bases and the fewest gaps. It allows for the provision of a gap creation penalty and a gap extension penalty in units of matched bases. GAP must make a profit of gap creation penalty number of matches for each gap it inserts. If a gap extension penalty greater than zero is chosen, GAP must, in addition, make a profit for each gap inserted of the length of the gap times the gap extension penalty. Default gap creation penalty values and gap extension penalty values in Version 10 of the Wisconsin Genetics Software Package for protein sequences are 8 and 2, respectively. For nucleotide sequences the default gap creation penalty is 50 while the default gap extension penalty is 3. The gap creation and gap extension penalties can be expressed as an integer selected from the group of integers consisting of from 0 to 200. Thus, for example, the gap creation and gap extension penalties can be 0,1, 2, 3, 4, 5, 6, 7, 8, 9, 10, 15, 20, 25, 30, 35, 40, 45, 50, 55, 60, 65 or greater.
   GAP presents one member of the family of best alignments. There may be many members of this family, but no other member has a better quality. GAP displays four figures of merit for alignments: Quality, Ratio, Identity, and Similarity. The Quality is the metric maximized in order to align the sequences. Ratio is the quality divided by the number of bases in the shorter segment. Percent Identity is the percent of the symbols that actually match. Percent Similarity is the percent of the symbols that are similar. Symbols that are across from gaps are ignored. A similarity is scored when the scoring matrix value for a pair of symbols is greater than or equal to 0.50, the similarity threshold. The scoring matrix used in Version 10 of the Wisconsin Genetics Software Package is BLOSUM62 (see Henikoff and Henikoff (1989) Proc. Natl. Acad. Sci. USA 89:10915).
(c) As used herein, "sequence identity" or "identity" in the context of two nucleic acid or polypeptide sequences makes reference to the residues in the two sequences that are the same when aligned for maximum correspondence over a specified comparison window. When percentage of sequence identity is used in reference to proteins it is recognized that residue positions which are not identical often differ by conservative amino acid substitutions, where amino acid residues are substituted for other amino acid residues with similar chemical properties (e.g., charge or hydrophobicity) and therefore do not change the functional properties of the molecule. When sequences differ in conservative substitutions, the percent sequence identity may be adjusted upwards to correct for the conservative nature of the substitution. Sequences that differ by such conservative substitutions are said to have "sequence similarity" or "similarity." Means for making this adjustment are well known to those of skill in the art. Typically this involves scoring a conservative substitution as a partial rather than a full mismatch, thereby increasing the percentage sequence identity. Thus, for example, where an identical amino acid is given a score of I and a non-conservative substitution is given a score of zero, a conservative substitution is given a score between zero and 1. The scoring of conservative substitutions is calculated, e.g., as implemented in the program PC/GENE (Intelligenetics, Mountain View, California).
(d) As used herein, "percentage of sequence identity" means the value determined by comparing two optimally aligned sequences over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions; dividing the number of matched positions by the total number of positions in the window of comparison, and multiplying the result by 100 to yield the percentage of sequence identity.
(e)
   (i) The term "substantial identity" of polynucleotide sequences means that a polynucleotide comprises a sequence that has at least 80% identity, more preferably at least 90%, and most preferably at least 95%, compared to a reference sequence using one of the alignment programs described using standard parameters. One of skill in the art will recognize that these values can be appropriately adjusted to determine corresponding identity of proteins encoded by two nucleotide sequences by taking into account codon degeneracy, amino acid similarity, reading frame positioning, and the like. Substantial identity of amino acid sequences for these purposes normally means sequence identity of at least 80%, 90%, and most preferably at least 95%.
      Another indication that nucleotide sequences are substantially identical is if two molecules hybridize to each other under stringent conditions. Generally, stringent conditions are selected to be about 5°C lower than the thermal melting point (Tₘ) for the specific sequence at a defined ionic strength and pH. However, stringent conditions encompass temperatures in the range of about 1°C to about 20°C lower than the Tₘ, depending upon the desired degree of stringency as otherwise qualified herein. Nucleic acids that do not hybridize to each other under stringent conditions are still substantially identical if the polypeptides they encode are substantially identical. This may occur, e.g., when a copy of a nucleic acid is created using the maximum codon degeneracy permitted by the genetic code. One indication that two nucleic acid sequences are substantially identical is when the polypeptide encoded by the first nucleic acid is immunologically cross reactive with the polypeptide encoded by the second nucleic acid.
   (e)(ii) The term "substantial identity" in the context of a peptide indicates that a peptide comprises a sequence with at least 80% sequence identity to a reference sequence, more preferably 85%, most preferably at least 90% or 95% sequence identity to the reference sequence over a specified comparison window. Preferably, optimal alignment is conducted using the homology alignment algorithm of Needleman et al. (1970) J. Mol. Biol. 48:443. An indication that two peptide sequences are substantially identical is that one peptide is immunologically reactive with antibodies raised against the second peptide. Thus, a peptide is substantially identical to a second peptide, for example, where the two peptides differ only by a conservative substitution. Peptides that are "substantially similar" share sequences as noted above except that residue positions that are not identical may differ by conservative amino acid changes.

The use of the term "nucleotide constructs" herein is not intended to limit the present invention to nucleotide constructs comprising DNA. Those of ordinary skill in the art will recognize that nucleotide constructs, particularly polynucleotides and oligonucleotides, comprised of ribonucleotides and combinations of ribonucleotides and deoxyribonucleotides may also be employed in the methods disclosed herein. Thus, the nucleotide constructs of the present invention encompass all nucleotide constructs that can be employed in the methods of the present invention for transforming plants including, but not limited to, those comprised of deoxyribonucleotides, ribonucleotides, and combinations thereof. Such deoxyribonucleotides and ribonucleotides include both naturally occurring molecules and synthetic analogues. The nucleotide constructs of the invention also encompass all forms of nucleotide constructs including, but not limited to, single-stranded forms, double-stranded forms, hairpins, stem-and-loop structures, and the like.

One may employ a nucleotide construct that is capable of directing, in a transformed plant, the expression of at least one protein, or at least one RNA, such as, for example, an antisense RNA that is complementary to at least a portion of an mRNA. Typically such a nucleotide construct is comprised of a coding sequence for a protein or an RNA operably linked to 5' and 3' transcriptional regulatory regions. Alternatively, it is also recognized that the methods of the invention may employ a nucleotide construct that is not capable of directing, in a transformed plant, the expression of a protein or an RNA.

In addition, it is recognized that methods of the present invention do not depend on the incorporation into the genome of the entire nucleotide construct comprising a P-glycoprotein nucleotide sequence, only that the plant or cell thereof is altered as a result of the introduction of the nucleotide construct into a cell. In one embodiment of the invention, the genome may be altered following the introduction of the nucleotide construct into a cell. For example, the nucleotide construct, or any part thereof, may incorporate into the genome of the plant. Alterations to the genome of the present invention include, but are not limited to, additions, deletions, and substitutions of nucleotides in the genome. While the methods of the present invention do not depend on additions, deletions, or substitutions of any particular number of nucleotides, it is recognized that such additions, deletions, or substitutions comprise at least one nucleotide.

The nucleotide constructs of the invention also encompass nucleotide constructs that may be employed in methods for altering or mutating a genomic nucleotide sequence in an organism, including, but not limited to, chimeric vectors, chimeric mutational vectors, chimeric repair vectors, mixed-duplex oligonucleotides, self-complementary chimeric oligonucleotides, and recombinogenic oligonucleobases. Such nucleotide constructs and methods of use, such as, for example, chimeraplasty, are known in the art. Chimeraplasty involves the use of such nucleotide constructs to introduce site-specific changes into the sequence of genomic DNA within an organism. See, U.S. Patent Nos. 5,565,350; 5,731,181; 5,756,325; 5,760,012; 5,795,972; and 5,871,984. See also, WO 98/49350, WO 99/07865, WO 99/25821, and Beetham et al. (1999) Proc. Natl. Acad Sci. USA 96:8774-8778.

The invention encompasses the use of methods, such as, for example, chimeraplasty to alter P-glycoprotein genes in plants. Such alterations include, for example, changes in the coding sequence that alter the amino acid sequence of the P-glycoprotein encoded thereby, resulting in a reduction in, or loss of, the function of the P-glycoprotein encoded by that gene.

The P-glycoprotein nucleotide sequences of the invention are provided in expression cassettes for expression in the plant of interest. The cassette will include 5'- and 3'-regulatory sequences operably linked to a P-glycoprotein nucleotide sequence of the invention. By "operably linked" is intended a functional linkage between a promoter and a second sequence, wherein the promoter sequence initiates and mediates transcription of the DNA sequence corresponding to the second sequence. Generally, operably linked means that the nucleic acid sequences being linked are contiguous and, where necessary to join two protein coding regions, contiguous and in the same reading frame. The cassette may additionally contain at least one additional gene to be cotransformed into the organism. Alternatively, the additional gene(s) can be provided on multiple expression cassettes.

Such an expression cassette is provided with a plurality of restriction sites for insertion of the P-glycoprotein nucleotide sequence to be under the transcriptional regulation of the regulatory regions. The expression cassette may additionally contain selectable marker genes.

The expression cassette will include in the 5'-3' direction of transcription, a transcriptional and translational initiation region, a P-glycoprotein nucleotide sequence of the invention, and a transcriptional and translational termination region functional in plants. The transcriptional initiation region, the promoter, may be native or analogous or foreign or heterologous to the plant host. Additionally, the promoter may be the natural sequence or alternatively a synthetic sequence. By "foreign" is intended that the transcriptional initiation region is not found in the native plant into which the transcriptional initiation region is introduced.

While it may be preferable to express the sequences using heterologous promoters, the native promoter sequences may be used. Such constructs would change expression levels of a P-glycoprotein in the plant or plant cell. Thus, the phenotype of the plant or plant cell is altered.

The termination region may be native with the transcriptional initiation region, may be native with the operably linked DNA sequence of interest, or may be derived from another source. Convenient termination regions are available from the Ti-plasmid of *A. tumefaciens,* such as the octopine synthase and nopaline synthase termination regions. See also Guerineau et al. (1991) Mol. Gen. Genet. 262:141-144*;* Proudfoot (1991) Cell 64:671-674; Sanfacon et al. (1991) Genes Dev. 5:141-149; Mogen et al. (1990) Plant Cell 2:1261-1272; Munroe et al. (1990) Gene 91:151-158; Ballas et al. (1989) Nucleic Acids Res. 17:7891-7903; and Joshi et al. (1987) Nucleic Acid Res. 15:9627-9639.

Where appropriate, the gene(s) may be optimized for increased expression in the transformed plant. That is, the genes can be synthesized using plant-preferred codons for improved expression. Methods are available in the art for synthesizing plant-preferred genes. See, for example, U.S. Patent Nos. 5,380,831, and 5,436,391, and Murray et al. (1989) Nucleic Acids Res. 17:477-498.

Additional sequence modifications are known to enhance gene expression in a cellular host. These include elimination of sequences encoding spurious polyadenylation signals, exon-intron splice site signals, transposon-like repeats, and other such well-characterized sequences that may be deleterious to gene expression. The G-C content of the sequence may be adjusted to levels average for a given cellular host, as calculated by reference to known genes expressed in the host cell. When possible, the sequence is modified to avoid predicted hairpin secondary mRNA structures.

The expression cassettes may additionally contain 5'-leader sequences in the expression cassette construct. Such leader sequences can act to enhance translation. Translation leaders are known in the art and include: picornavirus leaders, for example, EMC √ leader (Encephalomyocarditis 5'-noncoding region) (Elroy-Stein et al. (1989) PNAS USA 86:6126-6130); potyvirus leaders, for example, TEV leader (Tobacco Etch Virus) (Allison *et al.* (1986); MDMV leader (Maize Dwarf Mosaic Virus); Virology 154:9-20), and human immunoglobulin heavy-chain binding protein (BiP), (Macejak et al. (1991) Nature 353:90-94); untranslated leader from the coat protein mRNA of alfalfa mosaic virus (AMV RNA 4) (Jobling et al. (1987) Nature 325:622-625); tobacco mosaic virus leader (TMV) (Gallie et al. (1989) in Molecular Biology of RNA, ed. Cech (Liss, New York), pp. 237-256); and maize chlorotic mottle virus leader (MCMV) (Lommel et al. (1991) Virology 81:382-385). See also, Della-Cioppa et al. (1987) Plant Physiol. 84:965-968. Other methods known to enhance translation can also be utilized, for example, introns, and the like.

In preparing the expression cassette, the various DNA fragments may be manipulated, so as to provide for the DNA sequences in the proper orientation and, as appropriate, in the proper reading frame. Toward this end, adapters or linkers may be employed to join the DNA fragments or other manipulations may be involved to provide for convenient restriction sites, removal of superfluous DNA, removal of restriction sites, or the like. For this purpose, *in vitro* mutagenesis, primer repair, restriction, annealing, resubstitutions, e.g., transitions and transversions, may be involved.

It is recognized that with the nucleotide sequences of the invention, antisense constructions, complementary to at least a portion of the messenger RNA (mRNA) for the P-glycoprotein gene sequences can be constructed. Antisense nucleotides are constructed to hybridize with the corresponding mRNA. Modifications of the antisense sequences may be made as long as the sequences hybridize to and interfere with expression of the corresponding mRNA. In this manner, antisense constructions having 70%, preferably 80%, more preferably 85% sequence identity to the corresponding target sequences may be used. Furthermore, portions of the antisense nucleotides may be used to disrupt the expression of the target gene. Generally, sequences of at least 50 nucleotides, 100 nucleotides, 200 nucleotides, or greater may be used.

The nucleotide sequences of the present invention may also be used in the sense orientation to suppress the expression of endogenous genes in plants. Methods for suppressing gene expression in plants using nucleotide sequences in the sense orientation, also known as cosuppression methods, are known in the art. The methods generally involve transforming plants with a nucleotide construct comprising a promoter that drives expression in a plant operably linked to at least a portion of a nucleotide sequence that corresponds to the transcript of the endogenous gene. Typically, such a nucleotide sequence has substantial sequence identity to the sequence of the transcript of the endogenous gene, preferably greater than about 65% sequence identity, more preferably greater than about 85% sequence identity, most preferably greater than about 95% sequence identity. See, U.S. Patent Nos. 5,283,184 and 5,034,323.

Generally, the expression cassette will comprise a selectable marker gene for the selection of transformed cells. Selectable marker genes are utilized for the selection of transformed cells or tissues. Marker genes include genes encoding antibiotic resistance, such as those encoding neomycin phosphotransferase II (NEO) and hygromycin phosphotransferase (HPT), as well as genes conferring resistance to herbicidal compounds, such as glufosinate ammonium, bromoxynil, imidazolinones, and 2,4-dichlorophenoxyacetate (2,4-D). See generally, Yarranton (1992) Curr. Opin. Biotech. 3:506-511; Christopherson et al. (1992) Proc. Natl. Acad Sci. USA 89:6314-6318; Yao et al. (1992) Cell 71:63-72; Reznikoff (1992) Mol. Microbiol. 6:2419-2422; Barkley et al. (1980) in The Operon, pp. 177-220; Hu et al. (1987) Cell 48:555-566; Brown et al. (1987) Cell 49:603-612; Figge el al. (1988) Cell 52:713-722; Deuschle et al. (1989) Proc. Natl. Acad Aci. USA 86:5400-5404; Fuerst et al. (1989) Proc. Natl. Acad Sci. USA 86:2549-2553; Deuschle et al. (1990) Science 248:480-483; Gossen (1993) Ph.D. Thesis, University of Heidelberg; Reines et al. (1993) Proc. Natl. Acad Sci. USA 90:1917-1921; Labow et al. (1990) Mol. Cell. Biol. 10:3343-3356; Zambretti et al. (1992) Proc. Natl. Acad Sci. USA 89:3952-3956; Baim et al. (1991) Proc. Natl. Acad. Sci. USA 88:5072-5076; Wyborski et al. (1991) Nucleic Acids Res. 19:4647-4653; Hillenand-Wissman (1989) Topics Mol. Struc. Biol. 10:143-162; Degenkolb et al. (1991) Antimicrob. Agents Chemother. 35:1591-1595; Kleinschnidt et al. (1988) Biochemistry 27:1094-1104; Bonin (1993) Ph.D. Thesis, University of Heidelberg; Gossen et al. (1992) Proc. Natl. Acad. Sci. USA 89:5547-5551; Oliva et al. (1992) Antimicrob. Agents Chemother. 36:913-919; Hlavka et al. (1985) Handbook of Experimental Pharmacology, Vol. 78 ( Springer-Verlag, Berlin); Gill et al. (1988) Nature 334:721-724.

The above list of selectable marker genes is not meant to be limiting. Any selectable marker gene can be used in the present invention.

A number of promoters can be used in the practice of the invention. The promoters may be selected based on the desired timing, localization and level of expression of the P-glycoprotein genes in a plant. Constitutive, tissue-preferred, pathogen-inducible, wound-inducible and chemically regulatable promoters can be used in the practice of the invention.

Such constitutive promoters include, for example, the core promoter of the Rsyn7 promoter and other constitutive promoters disclosed in WO 99/43838 and U.S. Patent No. 6,072,050; the core CaMV 35S promoter (Odell et al. (1985) Nature 313:810-812); rice actin (McElroy et al. (1990) Plant Cell 2:163-171); ubiquitin (Christensen et al. (1989) Plant Mol. Biol. 12:619-632 and Christensen et al. (1992) Plant Mol. Biol. 18:675-689); pEMU (Last et al. (1991) Theor. Appl. Genet. 81:581-588); MAS (Velten et al. (1984) EMBO J. 3:2723-2730); ALS promoter (U.S. Application Serial No. 08/409,297), and the like. Other constitutive promoters include, for example, U.S. Patent Nos. 5,608,149; 5,608,144; 5,604,121; 5,569,597; 5,466,785; 5,399,680; 5,268,463; and 5,608,142.

Tissue-preferred promoters can be utilized to target enhanced P-glycoprotein expression within a particular plant tissue. Tissue-preferred promoters include Yamamoto et al. (1997) Plant J. 12(2):255-265; Kawamata et al. (1997) Plant Cell Physiol. 38(7):792-803; Hansen et al. (1997) Mol. Gen Genet. 254(3):337-343; Russell et al. (1997) Transgenic Res. 6(2):157-168; Rinehart et al. (1996) Plant Physiol. 112(3):1331-1341; Van Camp et al. (1996) Plant Physiol. 112(2):525-535; Canevascini et al. (1996) Plant Physiol. 112(2):513-524; Yamamoto et al. (1994) Plant Cell Physiol. 35(5):773-778; Lam (1994) Results Probl. Cell Differ. 20:181-196; Orozco et al. (1993) Plant Mol Biol. 23(6):1129-1138; Matsuoka et al. (1993) Proc Natl. Acad Sci. USA 90(20):9586-9590; and Guevara-Garcia et al. (1993) Plant J. 4(3):495-505. Such promoters can be modified, if necessary, for weak expression.

Leaf-preferred promoters include, Yamamoto et al. (1997) Plant J. 12(2):255-265; Kawamata et al. (1997) Plant Cell Physiol. 38(7):792-803; Hansen et al. (1997) Mol. Gen. Genet. 254(3):337-343; Russell et al. (1997) Transgenic Res. 6(2):157-168; Rinehart et al. (1996) Plant Physiol. 112(3):1331-1341; Van Camp et al. (1996) Plant Physiol. 112(2):525-535; Canevascini et al. (1996) Plant Physiol. 112(2):513-524; Yamamoto et al. (1994) Plant Cell Physiol. 35(5):773-778; Lam (1994) Results Probl. Cell Differ. 20:181-196; Orozco et al. (1993) Plant Mol. BioL 23(6):1129-1138; Matsuoka et al. (1993) Proc. Natl. Acad Sci. USA 90(20):9586-9590; and Guevara-Garcia et al. (1993) Plant J. 4(3):495-505.

Root-preferred promoters are known and can be selected from the many available from the literature or isolated de novo from various compatible species. See, for example, Hire et al. (1992) Plant Mol. Biol. 20(2): 207-218 (soybean root-preferred glutamine synthetase gene); Keller and Baumgartner (1991) Plant Cell 3(10):1051-1061 (root-preferred control element in the GRP 1.8 gene of French bean); Sanger et al. (1990) Plant Mol. Biol. 14(3):433-443 (root-preferred promoter of the mannopine synthase (MAS) gene of *Agrobacterium tumefaciens*); and Miao et al. (1991) Plant Cell 3(1):11-22 (full-length cDNA clone encoding cytosolic glutamine synthetase (GS), which is expressed in roots and root nodules of soybean). See also Bogusz et al. (1990) Plant Cell 2(7):633-641, where two root-preferred promoters isolated from hemoglobin genes from the nitrogen-fixing nonlegume Parasponia andersonii and the related non-nitrogen-fixing nonlegume Trema tomentosa are described. The promoters of these genes were linked to a β-glucuronidase reporter gene and introduced into both the nonlegume *Nicotiana tabacum* and the legume *Lotus corniculatus,* and in both instances root-preferred promoter activity was preserved. Leach and Aoyagi (1991) describe their analysis of the promoters of the highly expressed rolC and rolD root-inducing genes of *Agrobacterium rhizogenes* (see Plant Science (Limerick) 79(1):69-76). They concluded that enhancer and tissue-preferred DNA determinants are dissociated in those promoters. Teeri *et al.* (1989) used gene fusion to lacZ to show that the *Agrobacterium* T-DNA gene encoding octopine synthase is especially active in the epidermis of the root tip and that the TR2' gene is root preferred in the intact plant and stimulated by wounding in leaf tissue, an especially desirable combination of characteristics for use with an insecticidal or larvicidal gene (see EMBO J. 8(2):343-350)*.* The TR1' gene, fused to *nptII* (neomycin phosphotransferase II) showed similar characteristics. Additional root-preferred promoters include the VfENOD-GRP3 gene promoter (Kuster et al. (1995) Plant Mol. Biol. 29(4):759-772); and rolB promoter (Capana et al. (1994) Plant Mol. Biol. 25(4):681-691. See also U.S. Patent Nos. 5,837.876; 5,750,386; 5,633,363; 5,459,252; 5,401,836; 5,110,732; and 5,023,179.

Generally, it will be beneficial to express the gene from an inducible promoter, particularly from a pathogen-inducible promoter. Such promoters include those from pathogenesis-related proteins (PR proteins), which are induced following infection by a pathogen; e.g., PR proteins, SAR proteins, beta-1,3-glucanase, chitinase, etc. See, for example, Redolfi et al. (1983) Neth. J. Plant Pathol. 89:245-254; Uknes et al. (1992) Plant Cell 4:645-656; and Van Loon (1985) Plant Mol. Virol. 4:111-116. See also the copending applications entitled "Inducible Maize Promoters", U.S. Application Serial No. 60/076,100, filed February 26, 1998, and U.S. Application Serial No. 60/079,648, filed March 27, 1998, both of which correspond to PCT application WO 99/43819, published on 2 september 1999.

Of interest are promoters that are expressed locally at or near the site of pathogen infection. See, for example, Marineau et al. (1987) Plant Mol. Biol. 9:335-342; Matton et al. (1989) Molecular Plant-Microbe Interactions 2:325-331; Somsisch et al. (1986) Proc. Natl. Acad. Sci. USA 83:2427-2430; Somsisch et al. (1988) Mol. Gen. Genet. 2:93-98; and Yang (1996) Proc. Natl. Acad. Sci. USA 93:14972-14977. See also, Chen et al. (1996) Plant J. 10:955-966; Zhang et al. (1994) Proc. Natl. Acad. Sci. USA 91:2507-2511; Warner et al. (1993) Plant J. 3:191-201; Siebertz et al. (1989) Plant Cell 1:961-968; U.S. Patent No. 5,750,386 (nematode-inducible); and the references cited therein. Of particular interest is the inducible promoter for the maize PRms gene, whose expression is induced by the pathogen *Fusarium moniliforme* (see, for example, Cordero et al. (1992) Physiol. Mol. Plant Path. 41:189-200).

Additionally, as pathogens find entry into plants through wounds or insect damage, a wound-inducible promoter may be used in the constructions of the invention. Such wound-inducible promoters include potato proteinase inhibitor (pin II) gene (Ryan (1990) Ann. Rev. Phytopath. 28:425-449; Duan et al. (1996) Nature Biotechnology 14:494-498); wun1 and wun2, US Patent No. 5,428,148; win1 and win2 (Stanford et al. (1989) Mol. Gen. Genet. 215:200-208); systemin (McGurl et al. (1992) Science 225:1570-1573); WIP1 (Rohmeier et al. (1993) Plant Mol. Biol. 22:783-792; Eckelkamp et al. (1993) FEBS Letters 323:73-76); MPI gene (Corderok et al. (1994) Plant J 6(2):141-150).

Chemically regulated promoters can be used to modulate the expression of a gene in a plant through the application of an exogenous chemical regulator. Depending upon the objective, the promoter may be a chemical-inducible promoter, where application of the chemical induces gene expression, or a chemical-repressible promoter, where application of the chemical represses gene expression. Chemically inducible promoters are known in the art and include, but are not limited to, the maize In2-2 promoter, which is activated by benzenesulfonamide herbicide safeners, the maize GST promoter, which is activated by hydrophobic electrophilic compounds that are used as pre-emergent herbicides, and the tobacco PR-1a promoter, which is activated by salicylic acid. Other chemically regulated promoters of interest include steroid-responsive promoters (see, for example, the glucocorticoid-inducible promoter in Schena et al. (1991) Proc. Natl. Acad Sci. USA 88:10421-10425 and McNellis et al. (1998) Plant J 14(2):247-257) and tetracycline-inducible and tetracycline-repressible promoters (see, for example, Gatz et al. (1991) Mol. Gen. Genet. 227:229-237, and U.S. Patent Nos. 5,814,618 and 5,789,156).

Transformation protocols as well as protocols for introducing nucleotide sequences into plants may vary depending on the type of plant or plant cell, i.e., monocot or dicot, targeted for transformation. Suitable methods of introducing nucleotide sequences into plant cells and subsequent insertion into the plant genome include microinjection (Crossway et al. (1986) Biotechniques 4:320-334), electroporation (Riggs et al. (1986) Proc. Natl. Acad Sci. USA 83:5602-5606, Agrobacterium-mediated transformation (Townsend et al., U.S. Patent No. 5,563,055; Zhao et al., U.S. Patent No. 5,981,840), direct gene transfer (Paszkowski et al. (1984) EMBO J. 3:2717-2722), and ballistic particle acceleration (see, for example, Sanford et al., U.S. Patent No. 4,945,050; Tomes et al. (1995) "Direct DNA Transfer into Intact Plant Cells via Microprojectile Bombardment," in Plant Cell, Tissue, and Organ Culture: Fundamental Methods, ed. Gamborg and Phillips (Springer-Verlag, Berlin); and McCabe et al. (1988) Biotechnology 6:923-926). Also see Weissinger et al. (1988) Ann Rev. Genet. 22:421-477; Sanford et al. (1987) Particulate Science and Technology 5:27-37 (onion); Christou et al. (1988) Plant Physiol. 87:671-674 (soybean); McCabe et al. (1988) Bio/Technology 6:923-926 (soybean); Finer and McMullen (1991) In Vitro Cell Dev. BioL 27P:175-182 (soybean); Singh et al. (1998) Theor. Appl. Genet. 96:319-324 (soybean); Datta et al. (1990) Biotechnology 8:736-740 (rice); Klein et al. (1988) Proc. Natl. Acad Sci. USA 85:4305-4309 (maize); Klein et al. (1988) Biotechnology 6:559-563 (maize); Tomes, U.S. Patent No. 5,240,855; Buising et al., U.S. Patent Nos. 5,322,783 and 5,324,646; Tomes et al. (1995) "Direct DNA Transfer into Intact Plant Cells via Microprojectile Bombardment," in Plant Cell, Tissue, and Organ Culture: Fundamental Methods, ed. Gamborg (Springer-Verlag, Berlin) (maize); Klein et al. (1988) Plant Physiol. 91:440-444 (maize); Fromm et al. (1990) Biotechnology 8:833-839 (maize); Hooykaas-Van Slogteren et al. (1984) Nature (London) 311:763-764; Bytebier et al. (1987) Proc. Natl. Acad. Sci. USA 84:5345-5349 (Liliaceae); De Wet et al. (1985) in The Experimental Manipulation of Ovule Tissues, ed. Chapman et al. (Longman, New York), pp. 197-209 (pollen); Kaeppler et al. (1990) Plant Cell Reports 9:415-418 and Kaeppler et al. (1992) Theor. AppL Genet. 84:560-566 (whisker-mediated transformation); D'Halluin et al. (1992) Plant Cell 4:1495-1505 (electroporation); Li et al. (1993) Plant Cell Reports 12:250-255 and Christou and Ford (1995) Annals of Botany 75:407-413 (rice); Osjoda et al. (1996) Nature Biotechnology 14:745-750 (maize via *Agrobacterium tumefaciens*):

Alternatively, the nucleotide sequences of the invention can be introduced into an organism and allowed to undergo recombination with homologous regions of the organism's genome. Such homologous recombination approaches are well known to those of ordinary skill in the art and can be used to stably incorporate sequences of the invention into an organism. Further, such strategies can be used to introduce "knockout mutations" into a specific gene of an organism that shares substantial homology to the sequences of the invention. A knockout mutation is any mutation in the sequence of a gene that eliminates or substantially reduces the function or the level of the product encoded by the gene. Methods involving transformation of an organism followed by homologous recombination to stably integrate the sequences of the invention into the genome organism are encompassed by the invention. The invention is particularly directed to methods where sequences of the invention are utilized to alter the growth of an organism. Such methods encompass use of the sequences of the invention to interfere with the function or synthesis of a P-glycoprotein that controls growth of an organism.

The cells that have been transformed may be grown into plants in accordance with conventional ways. See, for example, McCormick et al. (1986) Plant Cell Reports 5:81-84. These plants may then be grown, and either pollinated with the same transformed strain or different strains, and the resulting hybrid having constitutive expression of the desired phenotypic characteristic identified. Two or more generations may be grown to ensure that constitutive expression of the desired phenotypic characteristic is stably maintained and inherited and then seeds harvested to ensure constitutive expression of the desired phenotypic characteristic has been achieved.

The present invention may be used for transformation of any plant species, including, but not limited to, corn (*Zea mays*), *Brassica* sp. (e.g., *B. napus, B. rapa, B. juncea),* particularly those *Brassica* species useful as sources of seed oil, alfalfa (*Medicago sativa*), rice (*Oryza sativa*), rye (*Secale cereale*), sorghum (*Sorghum bicolor, Sorghum vulgare*), millet (e.g., pearl millet *(Pennisetum glaucum),* proso millet (*Panicum miliaceum*), foxtail millet (*Setaria italica*), finger millet (*Eleusine coracana*)), sunflower (*Helianthus annuus*), safflower (*Carthamus tinctorius*), wheat *(Triticum aestivum),* soybean *(Glycine max),* tobacco *(Nicotiana tabacum),* potato (*Solanum tuberosum*), peanuts (*Arachis hypogaea*), cotton (*Gossypium barbadense, Gossypium hirsutum*), sweet potato (*Ipomoea batatus*), cassava (*Manihot esculenta*), coffee (*Coffea* spp.), coconut (*Cocos nucifera*), pineapple (*Ananas comosus*), citrus trees (*Citrus* spp.), cocoa (*Theobroma cacao*), tea (*Camellia sinensis*), banana (*Musa* spp.), avocado *(Persea americana),* fig *(Ficus casica),* guava *(Psidium guajava),* mango *(Mangifera indica),* olive *(Olea europaea),* papaya *(Carica papaya),* cashew *(Anacardium occidentale),* macadamia *(Macadamia integrifolia*), almond *(Prunus amygdalus*), sugar beets *(Beta vulgaris),* sugarcane *(Saccharum* spp.), oats, barley, vegetables, ornamentals, and conifers.

Vegetables include tomatoes *(Lycopersicon esculentum),* lettuce (e.g., *Lactuca sativa),* green beans (*Phaseolus vulgaris*), lima beans (*Phaseolus limensis*), peas *(Lathyrus* spp.), and members of the genus *Cucumis* such as cucumber *(C. sativus),* cantaloupe (*C. cantalupensis),* and musk melon (*C. melo).* Ornamentals include azalea *(Rhododendron* spp.), hydrangea *(Macrophylla hydrangea),* hibiscus *(Hibiscus rosasanensis),* roses *(Rosa* spp.), tulips *(Tulipa* spp.), daffodils *(Narcissus* spp.), petunias (*Petunia hybrida*), carnation (*Dianthus caryophyllus*)*,* poinsettia *(Euphorbia pulcherrima*)*,* and chrysanthemum. Conifers that may be employed in practicing the present invention include, for example, pines such as loblolly pine *(Pinus taeda*), slash pine *(Pinus elliotii*), ponderosa pine (*Pinus ponderosa*), lodgepole pine *(Pinus contorta*), and Monterey pine *(Pinus radiata*); Douglas-fir (*Pseudotsuga menziesii*); Western hemlock (*Tsuga canadensis*); Sitka spruce *(Picea glauca*); redwood *(Sequoia sempervirens*); true firs such as silver fir *(Abies amabilis)* and balsam fir *(Abies balsamea);* and cedars such as Western red cedar (*Thuja plicata*) and Alaska yellow-cedar (*Chamaecyparis nootkatensis*). Preferably, plants of the present invention are crop plants (for example, rice, corn, alfalfa, sunflower, *Brassica,* soybean, cotton, safflower, peanut, sorghum, wheat, millet, tobacco, etc.), more preferably corn, rice and sorghum plants.

The following examples are offered by way of illustration and not by way of limitation.

### EXAMPLE I

### Sorghum dwarfing gene, Dw3, encodes a P-glycoprotein homologue

It is well established that the sorghum dwarfing phenotype conferred by the *dw3* recessive mutation is unstable, although the mechanism responsible for its instability remains unknown. The *dw3* allele, referred to here as the reference allele (*dw3-ref*), reverts back to the wild-type form (conferring a tall phenotype) with a frequency of about 0.4 % to about 1 %. As a result, it is a commonplace to witness a number of tall sorghum plants in a field of *dw3* dwarfs. To determine if there is any relationship between the maize *br2* gene and the sorghum *dw3* gene, leaf samples were collected from 8 dwarf and 8 tall (revertant) plants; these were expected to be true *dw3* isogenics (identical throughout the genome except at the *dw3* locus). The DNA of these samples was extracted, digested with *Pst*I, and subjected to Southern blot analysis using a probe from the maize *Br2* gene. A clear and consistent DNA polymorphism was observed between the tall and dwarf plants, with the restriction fragment from the revertant allele being about 1.0 kb smaller that the *dw3-ref* allele.

Two conclusions were made from this result. First, the sorghum *Dw3* locus is structurally and functionally homologous to the maize *Br2* gene, suggesting that they may turn out to be true orthologs (i.e., derived from the same ancestral gene by vertical descent). Second, since all revertants had the same RFLP pattern, and that the size of the revertant allele was smaller than the mutant allele, the mutable *dw3-ref* allele was probably caused by an insertion. To address the latter interpretation, sorghum DNA in the vicinity of the *Br2*-detected polymorphism was subjected to PCR amplification using two oligonucleotide primers (SEQ ID NOS: 5-6) derived from the nucleotide sequence of the maize *Br2* gene.

PCR products were amplified from genomic DNA isolated from the tall revertants and dwarf plants with the *dw3-ref allele.* The PCR products were subsequently cloned and sequenced. The results obtained showed that a duplication of 882 bp had occurred in exon 5 of the *dw3* gene that led to the generation of the *dw3-ref* mutant allele (SEQ ID NO: 1). Thus, the *dw3* dwarf phenotype in sorghum is likely due to an insertion-induced mutation that occurred within the Dw3 allele to give rise to the *dw3-ref* allele. A partial sequence of the tall revertant allele, designated *Dw3-T* is disclosed in SEQ ID NO: 3. The duplication present in the *dw3-ref mutant* allele also seems to be responsible for the unstable nature of *dw3-ref.* By an undetermined mechanism, this duplication is removed in tall revertants of *dw3-ref.*

Comparison of the partial amino acid sequence of the protein encoded by *Dw3-T* (SEQ ID NO: 4) revealed that, like BR2, this protein belongs to the family of multidrug-resistance-like P-glycoproteins. Whereas it shows more than 96% amino acid identity with the maize pgp1 (the *Br2* gene), it exhibits 81% and 79% identity with P-glycoprotein genes of *Arabidopsis thaliana* and potato respectively.

Since the instability of the *dw3-ref* allele may result from some genetic recombination between two copies of the duplicated part of the gene, it might not always be precise. Some instances may occur where one or more extra base pairs may be left behind or deleted, leading in either case to a frame shift mutation. Such events are thus expected to generate new mutant alleles of *dw3* that are devoid of the duplication. And since the duplication seems to be responsible for the instability of *dw3-ref,* the new mutant alleles of *dw3* are expected to exhibit a stable dwarfing phenotype. Such stable dwarf alleles are highly desirable for breeding improved sorghum cultivars, as the instability of *dw3* has been a constant nemesis for breeders for enhancing the production of sorghum.

To identify a stable *dw3* allele, DNA was extracted from 200 dwarf sorghum plants and subjected to Southern blot analysis using a probe from the maize *Br2* gene. Two dwarf plants were identified that exhibited a restriction pattern that was different from the rest of the dwarf plants. Genomic DNA was isolated from one of these two dwarf plants and amplified using the oligonucleotide primers (SEQ ID NOS: 5-6) as described *supra.* The PCR product was cloned and sequenced. Comparison of the nucleotide sequence of the cloned PCR product (SEQ ID NO: 2) from this dwarf plant to the sequence of *dw3-ref* (*SEQ* ID NO: 1) revealed that the duplication present in *dw-3-ref* was lost. Thus, this dwarf plant possesses a new *dw3* allele, designated as *dw3-1.* Comparison of the nucleotide sequence of the *dw3-1* allele with the *Dw3-T* allele demonstrated that the new *dw3-1* allele has undergone minor changes.

To separate the new *dw3-1* allele from the parental *dw3-ref* allele, the dwarf plant possessing the *dw3-1* was self pollinated and seeds from plant were collected and planted. From the progeny, plants that were homozygous for *dw3-1* were identified by Southern blot analysis, and the homozygous plants are being propagated to develop stable dwarfing germplasm for sorghum. In addition, eight separate Pioneer proprietary sorghum inbreds are also being genotyped for the presence of new mutant derivatives of *dw3-ref.* The inbreds that were utilized are AGK1G, MK7G, MQC100G, ZYL24, YYU28W, CAJ14W, FYL14W, and YGC87W. They were selected on the basis of their reversion frequency, which was rated high, moderate or low. These inbreds were planted outdoors in Johnston, Iowa during the summer of 1999. Two hundred plants from each line were RFLP genotyped by digesting their DNA with PstI and hybridizing the resulting blots with a gene specific probe from the 3' end of the maize *br2* gene. Four stable homozygous dwarf plants were identified from YYU28W and ten such plants were identified from FYL14W. Seeds from these stable dwarf plants have been harvested. The progeny of these stable dwarf plants can be used directly for the production of high-yielding sorghum hybrids with the desired stable dwarf phenotype.

### EXAMPLE 2

### Nucleotide Sequence of a Dw3 Gene that Encodes a Functional Gene Product

In order to clone the entire sequence from both the functional (*Dw3*) and the mutant (*dw3*-ref) alleles of the *dw3* locus, a tall revertant plant and a dwarf sibling were selected from the inbred AGK1G. In this inbred line, tall plants appear at a frequency of 0.1- 0.4 %. The genotype of the tall revertant plant was expected to be heterozygous at the *dw3* locus but otherwise identical to its dwarf sibling throughout the genome. To confirm that the tall revertant was heterozygous at the *dw3* locus, DNA samples isolated from this plant and a number of dwarf siblings were characterized by Southern analysis using three probes representing the 5', middle and 3' parts of the maize *br2* gene. As expected, polymorphism between dwart siblings and the tall plant was localized only at the 3' end of *dw3*. This analysis allowed the identification of two EcoRI fragments from the tall revertant that when combined contained the entire *Dw3* allele. These were a 14kb EcoRI fragment that contained the 5' portion of the gene and an 8.1 kb fragment that contained the rest of the gene. A 9.0 kb fragment from the *dw3*-ref allele was determined to correspond to the 8.1 kb EcoRI fragment of the *Dw3* allele. Three size-selected libraries (containing the 14 kb/EcoRI and 8.1 kb/EcoRI fragments from the tall revertant and the 9.0 kb/EcoRI fragment from the dwarf sibling) were constructed in Lambda cloning vectors of Stratagene. The 14.0 kb /EcoRI fragment library was constructed in λ Dash II and was screened with a probe coming from the extreme 5' end of the maize *br2* gene. The other two libraries were prepared in λ ZapII and were screened with a probe from the 3'end of the maize *br2* gene. Positive clones were isolated and λ DNA was extracted for each of these clones. The *Dw3* and *dw3* genes were PCR amplified into four overlapping 0.5 kb, 2.4 kb, 3.0 kb, and 1.3 kb fragments using gene specific primers and λ DNA as a template. These PCR fragments were cloned in TOPO vector (Invitrogen). From the dwarf *dw3* clone of 9.0 kb, a unique 888 bp SacI fragment containing a part of the duplicated region was subcloned into pBSK+ vector (Stratagene).

DNA from at least two colonies of each PCR clone was sequenced using M13 forward, M13 reverse, and gene specific primers (GSPs). The 888 bp SacI fragment from the *dw3-ref* clone was sequenced by using T3 and T7 vector-specific primers alone. Sequence information, both from the extreme 5' and 3' ends of *Dw3* and *dw3* genes, was gathered by sequencing directly the λ DNA of both the 14.0 kb and 8.1 kb clones, using gene-specific primers. All of the sequence information was compiled and compared to reveal the cause of dwarfing in sorghum. A pairwise alignments between *Dw3* and *Br2* genes was done at the protein level by using Clustal W Program and at the nucleotide level by using BLAST Program of NCBL.

A polynucleotide of 6827 bp containing the full length *Dw3* gene was assembled and is presented in SEQ ID NO: 7. Structurally, the *Dw3* gene has five exons and four introns. The length of five exons, from exon 1 through exon 5, is 616 bp, 537 bp, 326 bp, 230 bp, and 2400 bp, respectively. Intron 1 is 165 bp (nucleotides 639-803 of SEQ ID NO: 7); intron 2 is 110 bp (nucleotides 1441-1550); intron 3 is 846 bp (nucleotides 1877-2722); and intron 4 is 1471 bp (nucleotides 2953-4423) in length. The intron/exon boundaries of *Dw3* are identical to that of the *br2* gene of maize. The predicted *Dw3*-cDNA is 4209 bp long from the start codon to the end of the termination codon (SEQ ID NO: 8) and is thus 28 bp longer than the analogous region of the *Br2*-cDNA. Similarly, the predicted protein encoded by *Dw3* is 1402 amino acids long (SEQ ID NO: 9), as compared to the 1394 amino acids predicted protein from *Br2* gene. Multiple alignment results show that overall *Dw3* is 92% and 91.8% identical to the maize *Br2* gene at the nucleotide level and at the amino acid level, respectively.

PCR analysis of the polymorphic region between *dw3* and *br2* had earlier suggested that a duplication of a part of exon 5 resulted in the *dw3*-ref dwarfing allelle of sorghum. To address if it was exclusively the reason for the mutant nature of the *dw3*-ref allele, the sequence of the *Dw3* allele from the tall revertant was compared with that of the *dw3*-ref allele. As shown previously, the difference was detected only in exon 5 between these alleles. In the mutant allele (*dw3*-ref, SEQ ID NO: 1), a stretch of 882 bp in exon 5 (from nucleotides 5650-6531 of SEQ ID NO: 7) is duplicated at the 6532 nucleotide position in the same direction. This duplication converted the 1312 bp PstI restriction fragment (from nucleotides 5463 bp to 6775 of SEQ ID NO: 7) in the functional *Dw3* allele to the 2194 bp PstI fragment found in the *dw3*-ref allele, and thus was the cause for the polymorphism between these two alleles. Since no other changes were found between these alleles, the results clearly implicate this duplication as the sole cause for creating the *dw3* dwarfing allele of sorghum. The addition of 882 bp to the cDNA will no doubt have a serious ramification for the structure and activity of the DW3 protein. These findings also show how the *dw3*-ref allele spontaneously corrects itself, every now and then, by getting rid of the duplication. The mechanism, by which this correction occurs, remains unknown, as does the mechanism by which the duplication occurred in the first place.

### Example 3

### Transformation of Maize by Particle Bombardment and Regeneration of Transgenic Plants

Immature maize embryos from greenhouse donor plants are bombarded with a plasmid containing a P-glycoprotein nucleotide sequence of the invention operably linked to a promoter that drives expression in a plant and the selectable marker gene PAT (Wohlleben et al. (1988) Gene 70:25-37), which confers resistance to the herbicide Bialaphos. Alternatively, the selectable marker gene is provided on a separate plasmid. Transformation is performed as follows. Media recipes follow below.

### Preparation of Target Tissue

The ears are husked and surface sterilized in 30% Clorox bleach plus 0.5% Micro detergent for 20 minutes, and rinsed two times with sterile water. The immature embryos are excised and placed embryo axis side down (scutellum side up), 25 embryos per plate, on 560Y medium for 4 hours and then aligned within the 2.5-cm target zone in preparation for bombardment.

### Preparation of DNA

A plasmid vector comprising the P-glycoprotein nucleotide sequence of the invention operably linked to the plant promoter of interest is made. This plasmid DNA plus plasmid DNA containing a PAT selectable marker is precipitated onto 1.1 µm (average diameter) tungsten pellets using a CaCl₂ precipitation procedure as follows:
100 µl prepared tungsten particles in water
10 µl (1 µg) DNA in Tris EDTA buffer (1 µg total DNA)
100 µl 2.5 M CaCl₂
10 µl 0.1 M spermidine

Each reagent is added sequentially to the tungsten particle suspension, while maintained on the multitube vortexer. The final mixture is sonicated briefly and allowed to incubate under constant vortexing for 10 minutes. After the precipitation period, the tubes are centrifuged briefly, liquid removed, washed with 500 ml 100% ethanol, and centrifuged for 30 seconds. Again the liquid is removed, and 105 µl 100% ethanol is added to the final tungsten particle pellet. For particle gun bombardment, the tungsten/DNA particles are briefly sonicated and 10 µl spotted onto the center of each macrocarrier and allowed to dry about 2 minutes before bombardment.

### Particle Gun Treatment

The sample plates are bombarded at level #4 in particle gun #HE34-1 or #HE34-2. All samples receive a single shot at 650 PSI, with a total of ten aliquots taken from each tube of prepared particles/DNA.

### Subsequent Treatment

Following bombardment, the embryos are kept on 560Y medium for 2 days, then transferred to 560R selection medium containing 3 mg/liter Bialaphos, and subcultured every 2 weeks. After approximately 10 weeks of selection, selection-resistant callus clones are transferred to 288J medium to initiate plant regeneration. Following somatic embryo maturation (2-4 weeks), well-developed somatic embryos are transferred to medium for germination and transferred to the lighted culture room. Approximately 7-10 days later, developing plantlets are transferred to 272V hormone-free medium in tubes for 7-10 days until plantlets are well established. Plants are then transferred to inserts in flats (equivalent to 2.5" pot) containing potting soil and grown for 1 week in a growth chamber, subsequently grown an additional 1-2 weeks in the greenhouse, then transferred to classic 600 pots (1.6 gallon) and grown to maturity. Plants are monitored and scored for dwarf phenotype or other phenotype associated with expression of the P-glycoprotein nucleotides sequence of the invention.

### Bombardment and Culture Media

Bombardment medium (560Y) comprises 4.0 g/l N6 basal salts (SIGMA C-1416), 1.0 ml/l Eriksson's Vitamin Mix (1000X SIGMA-1511), 0.5 mg/l thiamine HCl, 120.0 g/l sucrose, 1.0 mg/l2,4-D, and 2.88 g/l L-proline (brought to volume with D-I H₂0 following adjustment to pH 5.8 with KOH); 2.0 g/l Gelrite (added after bringing to volume with D-I H₂O); and 8.5 mg/l silver nitrate (added after sterilizing the medium and cooling to room temperature). Selection medium (560R) comprises 4.0 g/l N6 basal salts (SIGMA C-1416),1.0 ml/l Eriksson's Vitamin Mix (1000X SIGMA-1511), 0.5 mg/l thiamine HCl, 30.0 g/l sucrose, and 2.0 mg/l2,4-D (brought to volume with D-I H₂0 following adjustment to pH 5.8 with KOH); 3.0 g/l Gelrite (added after bringing to volume with D-I H₂0); and 0.85 mg/l silver nitrate and 3.0 mg/l bialaphos(both added after sterilizing the medium and cooling to room temperature).

Plant regeneration medium (288J) comprises 4.3 g/l MS salts (GIBCO 11117-074), 5.0 ml/l MS vitamins stock solution (0.100 g nicotinic acid, 0.02 g/l thiamine HCL, 0.10 g/l pyridoxine HCL, and 0.40 g/l glycine brought to volume with polished D-I H₂0) (Murashige and Skoog (1962) Physiol. Plant. 15:473), 100 mg/l myo-inositol, 0.5 mg/l zeatin, 60 g/l sucrose, and 1.0 ml/l of 0.1 mM abscisic acid (brought to volume with polished D-I H₂0 after adjusting to pH 5.6); 3.0 g/l Gelrite (added after bringing to volume with D-I H₂0); and 1.0 mg/l indoleacetic acid and 3.0 mg/l bialaphos (added after sterilizing the medium and cooling to 60°C). Hormone-free medium (272V) comprises 4.3 g/l MS salts (GIBCO 11117-074), 5.0 ml/l MS vitamins stock solution (0.100 g/l nicotinic acid, 0.02 g/l thiamine HCL, 0.10 g/l pyridoxine HCL, and 0.40 g/l glycine brought to volume with polished D-I H₂0), 0.1 g/l myo-inositol, and 40.0 g/l sucrose (brought to volume with polished D-I H₂0 after adjusting pH to 5.6); and 6 g/l bacto-agar (added after bringing to volume with polished D-I H₂0), sterilized and cooled to 60° C.

### Example 4

### Agrobacterium-Mediated Transformation of Maize and Regeneration of Transgenic Plants

For *Agrobacterium-mediated* transformation of maize with a P-glycoprotein nucleotide sequence of the invention, preferably the method of Zhao is employed (U.S. Patent No. 5,981,840, and PCT patent publication WO98/32326; the contents of which are hereby incorporated by reference). Briefly, immature embryos are isolated from maize and the embryos contacted with a suspension of *Agrobacterium,* where the bacteria are capable of transferring the P-glycoprotein nucleotide sequence of the invention to at least one cell of at least one of the immature embryos (step 1: the infection step). In this step the immature embryos are preferably immersed in an *Agrobacterium* suspension for the initiation of inoculation. The embryos are co-cultured for a time with the *Agrobacterium* (step 2: the co-cultivation step). Preferably the immature embryos are cultured on solid medium following the infection step. Following this co-cultivation period an optional "resting" step is contemplated. In this resting step, the embryos are incubated in the presence of at least one antibiotic known to inhibit the growth of *Agrobacterium* without the addition of a selective agent for plant transformants (step 3: resting step). Preferably the immature embryos are cultured on solid medium with antibiotic, but without a selecting agent, for elimination of *Agrobacterium* and for a resting phase for the infected cells. Next, inoculated embryos are cultured on medium containing a selective agent and growing transformed callus is recovered (step 4: the selection step). Preferably, the immature embryos are cultured on solid medium with a selective agent resulting in the selective growth of transformed cells. The callus is then regenerated into plants (step 5: the regeneration step), and preferably calli grown on selective medium are cultured on solid medium to regenerate the plants.

Although the foregoing invention has been described in some detail by way of illustration and example for purposes of clarity of understanding, it will be obvious that certain changes and modifications may be practiced within the scope of the appended claims.

## Claims

1. An isolated nucleic acid molecule comprising a nucleotide sequence selected from the group consisting of:
(a) a nucleotide sequence set forth in SEQ ID NO: 1;
(b) a nucleotide sequence set forth in SEQ ID NO: 2;
(c) a nucleotide sequence set forth in SEQ ID NO: 3;
(d) a nucleotide sequence set forth in SEQ ID NO: 7;
(e) a nucleotide sequence set forth in SEQ ID NO: 8;
(f) a nucleotide sequence consisting of at least 150 contiguous nucleotides of the nucleotide sequence set forth in any one of (a)-(e), wherein said nucleotide sequence encodes a P-glycoprotein that controls plant growth;
(g) a nucleotide sequence encoding the amino acid sequence set forth in SEQ ID NO: 9;
(h) a nucleotide sequence encoding at least 70 contiguous amino acids of the amino acid sequence set forth in SEQ ID NO: 9, wherein said nucleotide sequence encodes a P-glycoprotein that controls plant growth;
(i) a nucleotide sequence comprising at least 80% identity to the sequence set forth in SEQ ID NO: 7, wherein said nucleotide sequence encodes a P-glycoprotein that controls plant growth;
(j) a nucleotide sequence comprising at least 80% identity to the sequence set forth in SEQ ID NO: 8, wherein said nucleotide sequence encodes a P-glycoprotein that controls plant growth;
(k) a nucleotide sequence that is complementary to the nucleotide sequence of any one of (a)-(k); and
(l) a nucleotide sequence that hybridizes under stringent conditions to the nucleotide sequence any one of (a)-(e), or to a complementary sequence thereof, wherein said stringent conditions comprise hybridization in a solution comprising 50% formamide, 1 M NaCl, 1% SDS at 37°C and a wash in 0.1X SSC at 60°C, and said nucleotide sequence encodes a P-glycoprotein that controls plant growth.

2. An expression cassette comprising the nucleotide sequence of claim 1, said nucleotide sequence operably linked to a promoter that drives expression in a plant cell.

3. The expression cassette of claim 2, wherein said promoter is selected from the group consisting of tissue-preferred, constitutive, chemically regulatable, and pathogen-preferred promoters.

4. A plant having been stably transformed with the expression cassette of claim 2.

5. The plant of claim 4, wherein said promoter is selected from the group consisting of tissue-preferred, constitutive, chemically regulatable, and pathogen-preferred promoters.

6. The plant of claim 4, wherein said nucleotide sequence is operably linked to said promoter for the production of antisense transcripts.

7. The plant of claim 4, wherein said plant is a monocot.

8. The plant of claim 7, wherein said monocot is selected from the group consisting of maize, wheat, rice, Basmati rice, sorghum, rye, millet and barley.

9. The plant of claim 4, wherein said plant is a dicot.

10. The plant of claim 9, wherein said dicot is selected from the group consisting of soybeans, sunflowers, safflowers, alfalfa, Brassica sp., cotton, peanuts and fruit trees.

11. A seed of any one of the plants of claims 4-10, wherein said seed comprises said expression cassette.

12. A method for modifying the growth of a plant, said method comprising transforming a plant with a nucleotide sequence encoding a P-glycoprotein wherein said P-glycoprotein functions to control growth of an organism, said nucleotide sequence operably linked for the production of antisense transcripts to a promoter capable of driving the expression of said sequence in said plant; wherein said nucleotide sequence is as defined as claim 1.

13. The method of claim 12, wherein the height of said plant is reduced.

14. The method of claim 12, wherein said plant is a monocot.

15. The method of claim 12, wherein said monocot is selected from the group consisting of maize, wheat, rice, Basmati rice, sorghum, rye, millet and barley.

16. A plant cell having been stably transformed with the expression cassette of claim 2.

17. A method for identifying a sorghum plant having a stable dwarf phenotype comprising:
(a) obtaining a dwarf sorghum plant which is a descendent of an unstable *dw3* dwarf sorghum plant.
(b) analyzing the genomic DNA from said dwarf sorghum plant to determine if at least one copy of a *dw3* gene lacks an insertion, or at least a portion of said insertion;
(c) selecting said dwarf sorghum plant if said dwarf sorghum plant comprises in its genome at least one copy of said *dw3* gene lacking said insertion, or lacking said portion thereof.

18. The method of claim 17, wherein said insertion is a duplication or a transposon.

19. An isolated protein comprising a member selected from the group consisting of:
(a) a polypeptide comprising the amino acid sequence set forth in SEQ ID NO: 9;
(b) a polypeptide comprising at least 70 contiguous amino acids of the amino acid sequence set forth in SEQ ID NO: 9, wherein said polypeptide is a P-glycoprotein that controls plant growth;
(c) a polypeptide encoded by the nucleotide sequence set forth in SEQ ID NO: 8; and
(d) a polypeptide encoded by an amino acid sequence comprising at least 80% identity to the amino sequence of (a), wherein said polypeptide is a P-glycoprotein that controls plant growth.

## Patentansprüche

1. Isoliertes Nukleinsäure-Molekül, umfassend eine Nukleotidsequenz, ausgewählt aus der Gruppe, bestehend aus:
(a) einer Nukleotidsequenz, die in SEQ ID NO:1 angegeben ist;
(b) einer Nukleotidsequenz, die in SEQ ID NO:2 angegeben ist;
(c) einer Nukleotidsequenz, die in SEQ ID NO:3 angegeben ist;
(d) einer Nukleotidsequenz, die in SEQ ID NO:7 angegeben ist;
(e) einer Nukleotidsequenz, die in SEQ ID NO:8 angegeben ist;
(f) einer Nukleotidsequenz, die aus wenigstens 150 fortlaufenden Nukleotiden der Nukleotidsequenz, die in einem von (a)-(e) angegeben ist, besteht, wobei die Nukleotidsequenz für ein P-Glycoprotein codiert, das Pflanzenwachstum kontrolliert;
(g) einer Nukleotidsequenz, die für die in SEQ ID NO:9 angegebene Aminosäuresequenz codiert;
(h) einer Nukleotidsequenz, die für wenigstens 70 fortlaufender Aminosäuren der in SEQ ID NO:9 angegebenen Aminosäuresequenz codiert, wobei die Nukleotidsequenz für ein P-Glycoprotein codiert, das Pflanzenwachstum kontrolliert;
(i) einer Nukleotidsequenz, die wenigstens 80% Identität zu der in SEQ ID NO:7 angegebenen Sequenz umfasst, wobei die Nukleotidsequenz für ein P-Glycoprotein codiert, das Pflanzenwachstum kontrolliert;
(j) einer Nukleotidsequenz, die wenigstens 80% Identität zu der in SEQ ID NO:8 angegebenen Sequenz hat, wobei die Nukleotidsequenz für ein P-Glycoprotein codiert, das Pflanzenwachstum kontrolliert;
(k) einer Nukleotidsequenz, die zu der Nukleotidsequenz von einem von (a)-(k) komplementär ist, und
(l) einer Nukleotidsequenz, die unter stringenten Bedingungen an die Nukleotidsequenz von einem von (a)-(e) oder an eine komplementäre Sequenz derselben hybridisiert, wobei die stringenten Bedingungen eine Hybridisierung in einer Lösung, die 50% Formamid, 1 M NaCl, 1% SDS umfasst, bei 37°C und ein Waschen in 0,1 x SSC bei 60°C umfassen und wobei die Nukleotidsequenz für ein P-Glycoprotein codiert, das Pflanzenwachstum kontrolliert.

2. Expressionskassette, die die Nukleotidsequenz nach Anspruch 1 umfasst, wobei die Nukleotidsequenz funktionell mit einem Promotor verknüpft ist, der eine Expression in einer Pflanzenzelle steuert.

3. Expressionskassette nach Anspruch 2, wobei der Promotor ausgewählt ist aus der Gruppe, bestehend aus Gewebe-bevorzugten, konstitutiven, chemisch regulierbaren und Pathogen-bevorzugten Promotoren.

4. Pflanze, die stabil mit der Expressionskassette nach Anspruch 2 transformiert ist.

5. Pflanze nach Anspruch 4, wobei der Promotor ausgewählt ist aus der Gruppe, bestehend aus Gewebe-bevorzugten, konstitutiven, chemisch regulierbaren und Pathogen-bevorzugten Promotoren.

6. Pflanze nach Anspruch 4, wobei die Nukleotidsequenz funktionell mit dem Promotor für die Produktion von Antisense-Transkripten verknüpft ist.

7. Pflanze nach Anspruch 4, wobei die Pflanze eine Monokotyle ist.

8. Pflanze nach Anspruch 7, wobei die Monokotyle ausgewählt ist aus der Gruppe, bestehend aus Mais, Weizen, Reis, Basmati-Reis, Sorghum, Roggen, Hirse und Gerste.

9. Pflanze nach Anspruch 4, wobei die Pflanze eine Dikotyle ist.

10. Pflanze nach Anspruch 9, wobei die Dikotyle ausgewählt ist aus der Gruppe, bestehend aus Sojabohnen, Sonnenblumen, Saflor, Alfalfa, Brassica sp., Baumwolle, Erdnüssen und Obstbäumen.

11. Samen von einer der Pflanzen nach den Ansprüchen 4-10, wobei der Samen die Expressionskassette umfasst.

12. Verfahren zur Modifizierung des Wachstums einer Pflanze, wobei das Verfahren umfasst: Transformieren einer Pflanze mit einer Nukleotidsequenz, die für ein P-Glycoprotein codiert, wobei das P-Glycoprotein unter Kontrolle des Wachstums eines Organismus wirkt, wobei die Nukleotidsequenz funktionell für die Produktion von Antisense-Transkripten mit einem Promotor verknüpft ist, der fähig ist, die Expression der Sequenz in der Pflanze zu steuern, wobei die Nukleotidsequenz wie in Anspruch 1 definiert ist.

13. Verfahren nach Anspruch 12, wobei die Höhe der Pflanze verringert wird.

14. Verfahren nach Anspruch 12, wobei die Pflanze eine Monokotyle ist.

15. Verfahren nach Anspruch 12, wobei die Monokotyle ausgewählt ist aus der Gruppe, bestehend aus Mais, Weizen, Reis, Basmati-Reis, Sorghum, Roggen, Hirse und Gerste.

16. Pflanzenzelle, die stabil mit der Expressionskassette nach Anspruch 2 transformiert wurde.

17. Verfahren zur Identifizierung einer Sorghumpflanze, die einen stabilen Zwergphänotyp hat, umfassend:
(a) Erhalten einer Zwergsorghumpflanze, die ein Nachkomme einer instabilen *dw3*-Zwergsorghumpflanze ist;
(b) Analysieren der genomischen DNA aus der Zwergsorghumpflanze, um zu bestimmen, ob wenigstens einer Kopie eines *dw3*-Gens eine Insertion oder wenigstens ein Teil der Insertion fehlt;
(c) Selektieren der Zwergsorghumpflanze, wenn die Zwergsorghumpflanze in ihrem Genom wenigstens eine Kopie des *dw3*-Gens, dem die Insertion fehlt oder dem wenigstens ein Teil davon fehlt, umfasst.

18. Verfahren nach Anspruch 17, wobei die Insertion eine Duplikation oder ein Transposon ist.

19. Isoliertes Protein, umfassend ein Glied, ausgewählt aus der Gruppe, bestehend aus:
(a) einem Polypeptid, das die in SEQ ID NO:9 angegebene Aminosäuresequenz umfasst;
(b) einem Polypeptid, das wenigstens 70 fortlaufende Aminosäuren der in SEQ ID NO:9 angegebenen Aminosäuresequenz umfasst, wobei das Polypeptid ein P-Glycoprotein ist, das Pflanzenwachstum kontrolliert;
(c) einem Polypeptid, das durch die in SEQ ID NO:8 angegebene Nukleotidsequenz codiert wird; und
(d) einem Polypeptid, das durch eine Aminosäuresequenz codiert wird, die wenigstens 80% Identität zu der Aminosäuresequenz von (a) umfasst, wobei das Polypeptid ein P-Glycoprotein ist, das Pflanzenwachstum kontrolliert.

## Revendications

1. Molécule d'acide nucléique isolée comprenant une séquence nucléotidique choisie dans le groupe constitué de :
a) une séquence nucléotidique présentée dans la SEQ ID N° : 1 ;
b) une séquence nucléotidique présentée dans la SEQ ID N° : 2 ;
c) une séquence nucléotidique présentée dans la SEQ ID N° : 3 ;
d) une séquence nucléotidique présentée dans la SEQ ID N° : 7 ;
e) une séquence nucléotidique présentée dans la SEQ ID N° : 8 ;
f) une séquence nucléotidique constituée d'au moins 150 nucléotides contigus de la séquence nucléotidique présentée dans l'un quelconque des points a) à e), dans laquelle ladite séquence nucléotidique code une glycoprotéine P qui commande la croissance des végétaux ;
g) une séquence nucléotidique codant la séquence d'acides aminés présentée dans la SEQ ID N°: 9 ;
h) une séquence nucléotidique codant au moins 70 acides aminés contigus de la séquence d'acides aminés présentée dans la SEQ ID N°: 9, dans laquelle ladite séquence nucléotidique code une glycoprotéine P qui commande la croissance des végétaux ;
i) une séquence nucléotidique comprenant au moins 80% d'identité avec la séquence présentée dans la SEQ ID N°: 7, dans laquelle ladite séquence nucléotidique code une glycoprotéine P qui commande la croissance des végétaux ;
j) une séquence nucléotidique comprenant au moins 80% d'identité avec la séquence présentée dans la SEQ ID N°: 8, dans laquelle ladite séquence nucléotidique code une glycoprotéine P qui commande la croissance des végétaux ;
k) une séquence nucléotidique qui est complémentaire de la séquence nucléotidique de l'un quelconque des points a) à k) ; et
l) une séquence nucléotidique qui s'hybride dans des conditions stringentes à la séquence nucléotidique de l'un quelconque des points a) à e), ou à une séquence complémentaire de ces dernières, dans laquelle lesdites conditions stringentes comprennent l'hybridation dans une solution comprenant 50 % de formamide, 1 M de NaCl, 1 % de SDS à 37 °C et un lavage dans une solution 0,1X SSC à 60 °C, et ladite séquence nucléotidique code une glycoprotéine P qui commande la croissance des végétaux.

2. Cassette d'expression comprenant la séquence nucléotidique selon la revendication 1, ladite séquence nucléotidique étant liée de manière opérationnelle à un promoteur qui commande l'expression dans une cellule végétale.

3. Cassette d'expression selon la revendication 2, dans laquelle ledit promoteur est choisi dans le groupe constitué des promoteurs spécifiques des tissus, des promoteurs constitutifs, des promoteurs à régulation chimique, et des promoteurs spécifiques des pathogènes.

4. Végétal ayant été transformé de manière stable avec la cassette d'expression selon la revendication 2.

5. Végétal selon la revendication 4, dans lequel ledit promoteur est choisi dans le groupe constitué des promoteurs spécifiques des tissus, des promoteurs constitutifs, des promoteurs à régulation chimique, et des promoteurs spécifiques des pathogènes.

6. Végétal selon la revendication 4, dans lequel ladite séquence nucléotidique est liée de manière opérationnelle audit promoteur pour la production de transcrits antisens.

7. Végétal selon la revendication 4, dans lequel ledit végétal est une monocotylédone.

8. Végétal selon la revendication 7, dans lequel ladite monocotylédone est choisie dans le groupe constitué du maïs, du blé, du riz, du riz Basmati, du sorgho, du seigle, du mil et de l'orge.

9. Végétal selon la revendication 4, dans lequel ledit végétal est une dicotylédone.

10. Végétal selon la revendication 9, dans lequel ladite dicotylédone est choisie dans le groupe constitué du soja, du tournesol, du carthame, de la luzerne, des Brassica sp., du coton, des arachides, et des arbres fruitiers.

11. Graine de l'un quelconque des végétaux selon les revendications 4 à 10, dans laquelle ladite graine comprend ladite cassette d'expression.

12. Procédé de modification de la croissance d'un végétal, ledit procédé comprenant la transformation d'un végétal avec une séquence nucléotidique codant une glycoprotéine P dans lequel ladite glycoprotéine P fonctionne pour commander la croissance d'un organisme, ladite séquence nucléotidique étant liée de manière opérationnelle pour la production de transcrits antisens à un promoteur capable de commander l'expression de ladite séquence dans ledit végétal ; dans lequel ladite séquence nucléotidique est telle que définie dans la revendication 1.

13. Procédé selon la revendication 12, dans lequel la hauteur dudit végétal est réduite.

14. Procédé selon la revendication 12, dans lequel ledit végétal est une monocotylédone.

15. Procédé selon la revendication 12, dans lequel ladite monocotylédone est choisie dans le groupe constitué du maïs, du blé, du riz, du riz Basmati, du sorgho, du seigle, du mil et de l'orge.

16. Cellule végétale ayant été transformée de manière stable avec la cassette d'expression selon la revendication 2.

17. Procédé pour identifier du sorgho possédant un phénotype nain stable comprenant les étapes consistant à :
a) obtenir un sorgho nain qui est le descendant d'un sorgho nain instable *dw3*.
b) analyser l'ADN génomique à partir dudit sorgho nain pour déterminer s'il manque une insertion à au moins une copie d'un gène *dw3,* ou au moins une partie de ladite insertion ;
c) sélectionner ledit sorgho nain si ledit sorgho nain comprend dans son génome au moins une copie dudit gène *dw3* auquel il manque ladite insertion, ou ladite partie de cette dernière.

18. Procédé selon la revendication 17, dans lequel ladite insertion est une duplication ou un transposon.

19. Protéine isolée comprenant un élément choisi dans le groupe constitué de :
a) un polypeptide comprenant la séquence d'acides aminés présentée dans la SEQ ID N°. 9 ;
b) un polypeptide comprenant au moins 70 acides aminés contigus de la séquence d'acides aminés présentée dans la SEQ ID N°: 9, dans lequel ledit polypeptide est une glycoprotéine P qui commande la croissance des végétaux ;
c) un polypeptide codé par la séquence nucléotidique présentée dans la SEQ ID N°: 8; et
d) un polypeptide codé par une séquence d'acides aminés comprenant au moins 80 % d'identité avec la séquence d'acides aminés du point a), dans lequel ledit polypeptide est une glycoprotéine P qui commande la croissance des végétaux.
